# EUROPEAN PATENT APPLICATION

(11) **EP 3 495 818 A2**
(43) Date of publication of application: **12.06.2019**
(21) Application number: 18201180.9
(22) Date of filing: 12.04.2010
(51) Int. Cl.: G01N 33/543

(54) **IMPROVED METHODS FOR CONDUCTING ASSAYS**

(30) Priority: 10.04.2009 US 212377 P; 09.04.2010 US 757685
(62) Divisional of application: 10762560.0
(71) Applicant: Meso Scale Technologies, LLC, Gaithersburg, MD 20877 (US)
(72) Inventor: Glezer, Eli N., Chevy Chase, MD 20815 (US); Sigal, George, Rockville, MD 20853 (US); Tsionsky, Michael, Derwood, MD 20855 (US)
(74) Representative: Hoffmann Eitle

(57) **Abstract**

The invention relates to methods for conducting solid-phase binding assays. One example is an assay method having improved analyte specificity where specificity is limited by the presence of non-specific binding interactions.

## Description

### FIELD OF THE INVENTION

Improved methods for conducting binding assays are provided. These methods include a pre-concentration step to improve assay performance, for example, by increasing the concentration of analyte in the sample and/or by reducing the concentration of extraneous materials that may be present in the sample which may hinder the performance of the binding assay.

### BACKGROUND OF THE INVENTION

A substantial body of literature has been developed concerning techniques that employ binding reactions, e.g., antigen-antibody reactions, nucleic acid hybridization and receptor-ligand reactions, for the sensitive measurement of analytes of interest in samples. The high degree of specificity in many biochemical binding systems has led to many assay methods and systems of value in a variety of markets including basic research, human and veterinary diagnostics, environmental monitoring and industrial testing. The presence of an analyte of interest may be measured by directly measuring the participation of the analyte in a binding reaction. In some approaches, this participation may be indicated through the measurement of an observable label attached to one or more of the binding materials.

There is always a desire to improve binding assays by increasing the signal obtained from a binding event and/or improving measurement accuracy and precision, especially when analyzing complex biological samples.

### SUMMARY OF THE INVENTION

Thus, the present invention provides a method of conducting a binding assay comprising
(a) contacting (i) a sample comprising a target analyte with (ii) a particle linked to a first binding reagent that binds said target analyte and thereby forms a complex comprising said target analyte bound to said first binding reagent;
(b) collecting said complex;
(c) separating unbound components of said sample from said complex;
(d) releasing said complex;
(e) contacting said complex with a second binding reagent bound to a solid phase, wherein said second binding reagent binds to said complex; and
(f) measuring the amount of said analyte bound to said solid phase.

In an alternative embodiment, the invention provides a method of conducting a binding assay comprising
(a) contacting (i) a sample comprising a target analyte with (ii) a first solid phase linked to a first binding reagent that binds said target analyte and forms a complex comprising said target analyte bound to said first binding reagent;
(b) separating unbound components of said sample from said complex;
(c) releasing said complex;
(d) separating said complex from said first solid phase;
(e) contacting said complex with a second binding reagent bound to a second solid phase, wherein said second binding reagent binds to said complex; and
(f) measuring the amount of said analyte bound to said second solid phase.

Also provided is a method of conducting a binding assay comprising
(a) contacting (i) a sample comprising a target analyte with (ii) a particle linked to a first binding reagent that binds said target analyte and thereby forms a complex comprising said target analyte bound to said particle linked-first binding reagent;
(b) collecting said complex;
(c) separating unbound components of said sample from said complex;
(d) releasing said complex by cleaving said first binding reagent from said particle
(e) contacting said complex with a second binding reagent bound to a solid phase, wherein said second binding reagent binds to said complex;
(f) contacting said complex with a third binding reagent comprising a detectable label; and
(g) measuring the amount of said analyte bound to said solid phase.

Still further, the invention provides a method of conducting a binding assay comprising
(a) contacting (i) a sample comprising a target analyte with (ii) a particle linked to a first binding reagent that binds said target analyte and thereby forms a complex comprising said target analyte bound to said particle linked-first binding reagent;
(b) collecting said complex;
(c) separating unbound components of said sample from said complex;
(d) releasing said complex;
(e) contacting said complex with a second binding reagent bound to a solid phase, wherein said second binding reagent binds to said complex;
(g) contacting said complex with a third binding reagent comprising a detectable label; and
(f) measuring the amount of said analyte bound to said solid phase.

Also provided is a method of conducting a binding assay comprising
(a) contacting (i) a sample comprising a target analyte with (ii) a particle linked to a first binding reagent that binds said target analyte and thereby forms a complex comprising said target analyte bound to said particle linked-first binding reagent, wherein said particle comprises a detectable label;
(b) collecting said complex;
(c) separating unbound components of said sample from said complex;
(d) releasing said complex;
(e) contacting said complex with a second binding reagent bound to a solid phase, wherein said second binding reagent binds to said complex; and
(f) measuring the amount of said analyte bound to said solid phase.

Still further, the invention provides a method of conducting a binding assay comprising
(a) contacting (i) a sample comprising a target analyte with (ii) a particle linked to a first binding reagent that binds said target analyte and thereby forms a complex comprising said target analyte bound to said particle linked-first binding reagent, wherein said first binding reagent comprises a detectable label;
(b) collecting said complex;
(c) separating unbound components of said sample from said complex;
(d) releasing said complex;
(e) contacting said complex with a second binding reagent bound to a solid phase, wherein said second binding reagent binds to said complex; and
(f) measuring the amount of said analyte bound to said solid phase.

The invention also provides a method of conducting a binding assay for a plurality of analytes comprising
(a) contacting (i) a sample with (ii) one or more first solid phases linked to one or more first binding reagents that bind said analytes to form complexes comprising said analytes bound to said first binding reagents;
(b) separating unbound components of said sample from said complexes;
(c) releasing said complexes;
(d) contacting said complexes with a plurality of binding domains comprising second binding reagents that bind to said complexes, wherein each binding domain comprises a second binding reagent that binds to a complex comprising an analyte; and
(e) measuring the amount of said analytes bound to said binding domains.

In one embodiment of this method, at least two of said binding domains differ in the binding reagents comprised therein and differ in their selectivity for the complexes comprising at least two of said analytes.

Moreover, the invention provides a method of conducting a binding assay comprising
(a) contacting (i) a sample comprising a target analyte with (ii) a particle linked to a first binding reagent that binds said target analyte to form a complex comprising said target analyte bound to said first binding reagent, wherein said first binding reagent is linked to a first targeting agent and said particle is linked to a second targeting agent, and said first binding reagent and said particle are linked via a binding reaction between said first and second targeting agents;
(b) collecting said complex;
(c) separating unbound components of said sample from said complex;
(d) releasing said complex;
(e) contacting said complex with a second binding reagent bound to a solid phase, wherein said second binding reagent binds to said complex; and
(f) measuring the amount of said analyte bound to said solid phase,

Alternatively, the invention provides a method of conducting a binding assay comprising
(a) contacting (i) a sample comprising a target analyte with (ii) a first solid phase linked to a first binding reagent that binds said target analyte to form a complex comprising said target analyte bound to said first binding reagent, wherein said first binding reagent is linked to a first targeting agent and said first solid phase is linked to a second targeting agent, and said first binding reagent and said first solid phase are linked via a binding reaction between said first and second targeting agents;
(b) separating unbound components of said sample from said complex;
(c) releasing said complex;
(d) separating said complex from said first solid phase;
(e) contacting said complex with a second binding reagent bound to a second solid phase, wherein said second binding reagent binds to said complex; and
(f) measuring the amount of said analyte bound to said second solid phase.

Further provided is a method of conducting a binding assay comprising
(a) contacting (i) a sample comprising a target analyte with (ii) a particle linked to a first binding reagent that binds said target analyte to form a complex comprising said target analyte bound to said first binding reagent, wherein said first binding reagent is linked to a first targeting agent and said particle is linked to a second targeting agent, and said first binding reagent and said particle are linked via a binding reaction between said first and second targeting agents;
(b) collecting said complex;
(c) separating unbound components of said sample from said complex;
(d) releasing said complex by cleaving said first binding reagent from said particle;
(e) contacting said complex with a second binding reagent bound to a solid phase, wherein said second binding reagent binds to said complex;
(f) contacting said complex with a third binding reagent comprising a detectable label; and
(g) measuring the amount of said analyte bound to said solid phase.

Still further, the invention provides a method of conducting a binding assay comprising
(a) contacting (i) a sample comprising a target analyte with (ii) a particle linked to a first binding reagent that binds said target analyte to form a complex comprising said target analyte bound to said first binding reagent, wherein said first binding reagent is linked to a first targeting agent and said particle is linked to a second targeting agent, and said first binding reagent and said particle are linked via a binding reaction between said first and second targeting agents;
(b) collecting said complex;
(c) separating unbound components of said sample from said complex;
(d) releasing said complex;
(e) contacting said complex with a second binding reagent bound to a solid phase, wherein said second binding reagent binds to said complex;
(g) contacting said complex with a third binding reagent comprising a detectable label; and
(f) measuring the amount of said analyte bound to said solid phase.

The invention also provides a method of conducting a binding assay for a plurality of analytes comprising
(a) contacting (i) a sample , with (ii) one or more first solid phases linked to one or more first binding reagents that bind said analytes to form complexes comprising said analytes bound to said first binding reagents, wherein said first binding reagents are linked to first targeting agents and said first solid phases are linked to second targeting agents, and said first binding reagents and said first solid phases are linked via binding reactions between said first and second targeting agents;
(b) separating unbound components of said sample from said complexes;
(c) releasing said complexes;
(d) removing said first solid phase;
(e) contacting said complexes with a plurality of binding domains comprising second binding reagents that bind to said complexes, wherein each binding domain comprises a second binding reagent that binds to a complex comprising a secondary target analyte; and
(f) measuring the amount of said analytes bound to said binding domains.

In one embodiment of this method, at least two of said binding domains differ in the binding reagents comprised therein and differ in their selectivity for the complexes comprising at least two of said analytes.

In each of these embodiments, the releasing step may comprise resuspending said complex, and/or the releasing may comprise cleaving said binding reagent from the particle or other solid phase. Such cleaving step optionally comprises subjecting the complex to increased or decreased temperature, pH changes, competition, and combinations thereof. The collecting step may comprise a method selected from the group consisting of centrifugation, gravity, filtration, magnetic collection, and combinations thereof. The measuring step may comprise measuring optical absorbance, fluorescence, phosphorescence, chemiluminescence, light scattering or magnetism.

### BRIEF DESCRIPTION OF DRAWINGS

The accompanying drawings are provided to illustrate rather than limit the scope of the invention. Throughout the accompanying Figures, "P" refers to a particle to which one or more moieties are attached; "S" refers to a first solid phase; "A" refers to a target analyte; "C" refers to contaminants; and "*" refers to a detectable label linked to an assay component.
Figs. 1(a)-1(e) illustrate various assay formats in which a particle is used as an assay component.
Figs. 2(a)-2(b) illustrate various assay formats in which a first solid phase is used as an assay component.
Figs. 3(a)-3(e) illustrate various assay formats in which a particle is used as an assay component, to which a targeting agent is linked.
Figs. 4(a)-4(b) illustrate various assay formats in which a first solid phase is used as an assay component, to which a targeting agent is linked.
Figs. 5(a)-5(b) illustrates one embodiment of the present invention. Fig. 5(a) shows magnetic concentration of analytes using colloids coated with anti-antibodies against the analytes and also coated with ECL labels. Multiple antibodies may be used to bind different analytes. Fig. 5(b) shows detection of the analyte-colloid complexes in a sandwich immunoassay format.
Figs. 6(a)-6(b) illustrate two alternative competitive immunoassays according to the methods of the present invention.

### DETAILED DESCRIPTION OF THE INVENTION

The present invention provides improved solid phase binding assays that include a collection, separation and/or release step. The methods of the present invention improve assay performance by allowing for pre-concentration of an analyte in a sample and/or by reducing or eliminating the amount of contaminants in a sample that may hinder the performance of the assay, e.g., by interfering with the detection step and/or by non-specifically binding with one or more of the components in the mixture.

### (i) Samples/Analytes

Examples of samples that may be analyzed by the methods of the present invention include, but are not limited to food samples (including food extracts, food homogenates, beverages, etc.), environmental samples (e.g., soil samples, environmental sludges, collected environmental aerosols, environmental wipes, water filtrates, etc,), industrial samples (e.g., starting materials, products or intermediates from an industrial production process), human clinical samples, veterinary samples and other samples of biological origin. Biological samples that may be analyzed include, but are not limited to, feces, mucosal swabs, physiological fluids and/or samples containing suspensions of cells. Specific examples of biological samples include blood, serum, plasma, feces, mucosal swabs, tissue aspirates, tissue homogenates, cell cultures and cell culture supernatants (including cultures of eukaryotic and prokaryotic cells), urine, saliva, sputum, and cerebrospinal fluid.

Analytes that may be measured using the methods of the invention include, but are not limited to proteins, toxins, nucleic acids, microorganisms, viruses, cells, fungi, spores, carbohydrates, lipids, glycoproteins, lipoproteins, polysaccharides, drugs, hormones, steroids, nutrients, metabolites and any modified derivative of the above molecules, or any complex comprising one or more of the above molecules or combinations thereof. The level of an analyte of interest in a sample may be indicative of a disease or disease condition or it may simply indicate whether the patient was exposed to that analyte.

The assays of the present invention may be used to determine the concentration of one or more, e.g., two or more analytes in a sample. Thus, two or more analytes may be measured in the same sample. Panels of analytes that can be measured in the same sample include, for example, panels of assays for analytes or activities associated with a disease state or physiological conditions. Certain such panels include panels of cytokines and/or their receptors (e.g., one or more of TNF-alpha, TNF-beta, ILl-alpha, ILI-beta, IL2, IL4, IL6, IL-10, IL-12, IFN-y, etc.), growth factors and/or their receptors (e.g., one or more of EGF, VGF, TGF, VEGF, etc.), drugs of abuse, therapeutic drugs, vitamins, pathogen specific antibodies, auto-antibodies (e.g., one or more antibodies directed against the Sm, RNP, SS-A, SS-alpha, J0-1, and Scl-70 antigens), allergen-specific antibodies, tumor markers (e.g., one or more of CEA, PSA, CA-125 II, CA 15-3, CA 19-9, CA 72-4, CYFRA 21-1, NSE, AFP, etc.), markers of cardiac disease including congestive heart disease and/or acute myocardial infarction (e.g., one or more of Troponin T, Troponin I, myoglobin, CKMB, myeloperoxidase, glutathione peroxidase, β-natriuretic protein (BNP), alpha-natriuretic protein (ANP), endothelin, aldosterone, C-reactive protein (CRP), etc.), markers associated with hemostasis (e.g., one or more of Fibrin monomer, D-dimer, thrombin-antithrombin complex, prothrombin fragments 1 & 2, anti-Factor Xa, etc.), markers of acute viral hepatitis infection (e.g., one or more of IgM antibody to hepatitis A virus, IgM antibody to hepatitis B core antigen, hepatitis B surface antigen, antibody to hepatitis C virus, etc.), markers of Alzheimers Disease (alpha-amyloid, beta-amyloid, Aβ 42, Aβ 40, Aβ 38, Aβ 39, Aβ 37, Aβ 34, tau-protein, etc.), markers of osteoporosis (e.g., one or more of cross-linked Nor C-telopeptides, total deoxypyridinoline, free deoxypyridinoline, osteocalcin, alkaline phosphatase, C-terminal propeptide of type I collagen, bone-specific alkaline phosphatase, etc.), markers of fertility state or fertility associated disorders (e.g., one or more of Estradiol, progesterone, follicle stimulating hormone (FSH), lutenizing hormone (LH), prolactin, hCG, testosterone, etc.), markers of thyroid disorders (e.g., one or more of thyroid stimulating hormone (TSH), Total T3, Free T3, Total T4, Free T4, and reverse T3), and markers of prostrate cancer (e.g., one or more of total PSA, free PSA, complexed PSA, prostatic acid phosphatase, creatine kinase, etc.). Certain embodiments of invention include measuring, e.g., one or more, two or more, four or more or 10 or more analytes associated with a specific disease state or physiological condition (e.g., analytes grouped together in a panel, such as those listed above; e.g., a panel useful for the diagnosis of thyroid disorders may include e.g., one or more of thyroid stimulating hormone (TSH), Total T3, Free T3, Total T4, Free T4, and reverse T3).

The methods of the present invention are designed to allow detection of a wide variety of biological and biochemical agents, as described above. In one embodiment, the methods may be used to detect pathogenic and/or potentially pathogenic virus, bacteria and toxins including biological warfare agents ("BWAs") in a variety of relevant clinical and environmental matrices, including and without limitation, blood, sputum, stool, filters, swabs, etc. A non-limiting list of pathogens and toxins that may be analyzed (alone or in combination) using the methods of the present invention is *Bacillus anthracis* (anthrax), *Yersinia pestis* (plague), *Vibrio cholerae* (cholera), *Francisella tularensis* (tularemia), *Brucella* spp. (Brucellosis), *Coxiella burnetii* (Q fever), orthopox viruses including variola virus (smallpox), viral encephalitis, Venezuelan equine encephalitis virus (VEE), western equine encephalitis virus (WEE), eastern equine encephalitis virus (EEE), Alphavirus, viral hemorrhagic fevers, Arenaviridae, Bunyaviridae, Filoviridae, Flaviviridae, Ebola virus, staphylococcal enterotoxins, ricin, botulinum toxins, *Clostridium botulinum,* mycotoxin, *Fusarium, Myrotecium, Cephalosporium, Trichoderma, Verticimonosporium, Stachybotrys.* glanders, wheat fungus, *Bacillus globigii, Serratia marcescens,* yellow rain, trichothecene mycotoxins, *Salmonella typhimurium,* aflatoxin, *Xenopsylla cheopis, Diamanus montanus,* alastrim, monkeypox, Arenavirus, Hantavirus, Lassa fever, Argentine hemorrhagic fevers, Bolivian hemorrhagic fevers, Rift Valley fever virus, Crimean-Congo virus, Hanta virus, Marburg hemorrhagic fevers, yellow fever virus, dengue fever viruses, influenza (including human and animal strains including H5N1 avian influenza), human immunodeficiency viruses I and II (HIV I and II), hepatitis A, hepatitis B, hepatitis C, hepatitis (non-A, B or C), Enterovirus, Epstein-Barr virus, Cytomegalovirus, herpes simplex viruses, *Chlamydia trachomatis, Neisseria gonorrheae, Trichomonas vaginalis,* human papilloma virus, *Treponema pallidum, Streptococcus pneumonia, Haemophilus influenzae, Mycoplasma pneumoniae, Chlamydophila pneumoniae, Legionella pneumophila, Staphylococcus aureus, Moraxella catarrhalis, Streptococcus pyogenes, Clostridium difficile, Neisseria meningitidis, Klebsiella pneumoniae, Mycobacterium tuberculosis,* coronavirus, Coxsackie A virus, rhinovirus, parainfluenza virus, respiratory syncytial virus (RSV), metapneumovirus, and adenovirus.

### (ii) Binding Reagents

The skilled artisan in the field of binding assays will readily appreciate the scope of binding agents and companion binding partners that may be used in the present methods. A non-limiting list of such pairs include (in either order) oligonucleotides and complements, receptor/ligand pairs, antibodies/antigens, natural or synthetic receptor/ligand pairs, amines and carbonyl compounds (i.e., binding through the formation of a Schiff's base), hapten/antibody pairs, antigen/antibody pairs, epitope/antibody pairs, mimitope/antibody pairs, aptamer/target molecule pairs, hybridization partners, and intercalater/target molecule pairs.

The binding assays of the methods of the present invention may employ antibodies or other receptor proteins as binding reagents. The term "antibody" includes intact antibody molecules (including hybrid antibodies assembled by in vitro reassociation of antibody subunits), antibody fragments and recombinant protein constructs comprising an antigen binding domain of an antibody (as described, e.g., in Porter, R. R. and Weir, R. C. J Cell Physiol., 67 (Suppl); 51-64 (1966) and Hochman, 1. Inbar, D. and Givol, D. Biochemistry 12: 1130 (1973)), as well as antibody constructs that have been chemically modified, e.g., by the introduction of a detectable label.

Binding reagents and binding partners that are linked to assay components to enable the attachment of these assay components to each other or to solid phases may be described herein as "targeting agents". For targeting agents that work in pairs, e.g., antigen-antibody, oligonucleotides-complement, etc., one targeting agent of the pair may be referred to herein as the first targeting agent, whereas the companion targeting agent may be referred to as the second targeting agent. In certain embodiments, these targeting agents are selected based on the reversibility of their binding reactions. In particular, targeting agent pairs may be selected, e.g., because under a first set of conditions the pair will bind to form a binding complex which, under a second set of conditions, can be caused to dissociate to break apart the complex, e.g, by subjecting bound targeting agent pairs to increased or decreased temperature, changes in chemical environment or assay buffer (e.g., ionic strength changes, pH changes, addition of denaturants, etc.), adding competing binding reagents that compete with one targeting agent for binding to another targeting agent, and combinations thereof. Suitable conditions may be derived through routine experimentation. There are many well-established cleavable chemical linkers that may be used that provide a covalent bond that may be cleaved without requiring harsh conditions. For example, disulfide containing linkers may be cleaved using thiols or other reducing agents, cis-diol containing linkers may be cleaved using periodate, metal-ligand interactions (such as nickel-histidine) may be cleaved by changing pH or introducing competing ligands. The terms "cleave" or "cleaving" are also used herein to refer to processes for separating linked assay components that do not require breaking covalent bonds, e.g., there are many well-established reversible binding pairs and conditions that may be employed (including those that have been identified in the art of affinity chromatography). By way of example, the binding of many antibody-ligand pairs can be reversed through changes in pH, addition of protein denaturants or chaotropic agents, addition of competing ligands, etc.

The targeting agents may be pairs of oligonucleotides comprising complementary sequences. The preferred length is approximately 5 to 100 bases, preferably, approximately, 10 to 50 bases, and more preferably approximately 10 to 25 bases. In addition, the targeting oligonucleotides sequences need not be identical in length and in certain embodiments it may be beneficial to provide one targeting oligonucleotide sequence that is longer than its binding partner, e.g., by up to 25 bases, or up to 15 bases, or up to 10 bases. Known methods that are commonly employed for strand separation employ i) temperatures above the melting temperature for the complex, ii) use an alkaline pH of 11 (or higher) or a low pH; iii) use high ionic strength and/or iv) use nucleic acid denaturants such as formamide. Other methods for strand separation include the use of helicase enzymes such as Rep protein of *E.coli* that can catalyse the unwinding of the DNA, or binding proteins such as 32-protein of *E.coli* phage T4 that act to stabilize the single-stranded form of DNA. In specific embodiments, dissociation of complementary nucleic acid strands is accomplished by exposing the strands to elevated temperature greater than 60°C.

The methods of the present invention may be used in a variety of assay devices and/or formats. The assay devices may include, e.g., assay modules, such as assay plates, cartridges, multi-well assay plates, reaction vessels, test tubes, cuvettes, flow cells, assay chips, lateral flow devices, etc., having assay reagents (which may include targeting agents or other binding reagents) added as the assay progresses or pre-loaded in the wells, chambers, or assay regions of the assay module. These devices may employ a variety of assay formats for specific binding assays, e.g., immunoassay or immunochromatographic assays. Illustrative assay devices and formats are described herein below. In certain embodiments, the methods of the present invention may employ assay reagents that are stored in a dry state and the assay devices/kits may further comprise or be supplied with desiccant materials for maintaining the assay reagents in a dry state. The assay devices preloaded with the assay reagents can greatly improve the speed and reduce the complexity of assay measurements while maintaining excellent stability during storage. The dried assay reagents may be any assay reagent that can be dried and then reconstituted prior to use in an assay. These include, but are not limited to, binding reagents useful in binding assays, enzymes, enzyme substrates, indicator dyes and other reactive compounds that may be used to detect an analyte of interest. The assay reagents may also include substances that are not directly involved in the mechanism of detection but play an auxiliary role in an assay including, but not limited to, blocking agents, stabilizing agents, detergents, salts, pH buffers, preservatives, etc. Reagents may be present in free form or supported on solid phases including the surfaces of compartments (e.g., chambers, channels, flow cells, wells, etc.) in the assay modules or the surfaces of colloids, beads, or other particulate supports.

### (iii) Solid Phases

A wide variety of solid phases are suitable for use in the methods of the present invention including conventional solid phases from the art of binding assays. Solid phases may be made from a variety of different materials including polymers (e.g., polystyrene and polypropylene), ceramics, glass, composite materials (e.g., carbon-polymer composites such as carbon-based inks). Suitable solid phases include the surfaces of macroscopic objects such as an interior surface of an assay container (e.g., test tubes, cuvettes, flow cells, cartridges, wells in a multi-well plate, etc.), slides, assay chips (such as those used in gene or protein chip measurements), pins or probes, beads, filtration media, lateral flow media (for example, filtration membranes used in lateral flow test strips), etc.

Suitable solid phases also include particles (including but not limited to colloids or beads) commonly used in other types of particle-based assays e.g., magnetic, polypropylene, and latex particles, materials typically used in solid-phase synthesis e.g., polystyrene and polyacrylamide particles, and materials typically used in chromatographic applications e.g., silica, alumina, polyacrylamide, polystyrene. The materials may also be a fiber such as a carbon fibril. Microparticles may be inanimate or alternatively, may include animate biological entities such as cells, viruses, bacterium and the like.

The particles used in the present method may be comprised of any material suitable for attachment to one or more binding partners and/or labels, and that may be collected via, e.g., centrifugation, gravity, filtration or magnetic collection. A wide variety of different types of particles that may be attached to binding reagents are sold commercially for use in binding assays. These include non-magnetic particles as well as particles comprising magnetizable materials which allow the particles to be collected with a magnetic field. In one embodiment, the particles are comprised of a conductive and/or semiconductive material, e.g., colloidal gold particles.

The microparticles may have a wide variety of sizes and shapes. By way of example and not limitation, microparticles may be between 5 nanometers and 100 micrometers. Preferably microparticles have sizes between 20 nm and 10 micrometers. The particles may be spherical, oblong, rod-like, etc., or they may be irregular in shape.

The particles used in the present method may be coded to allow for the identification of specific particles or subpopulations of particles in a mixture of particles. The use of such coded particles has been used to enable multiplexing of assays employing particles as solid phase supports for binding assays. In one approach, particles are manufactured to include one or more fluorescent dyes and specific populations of particles are identified based on the intensity and/or relative intensity of fluorescence emissions at one or more wave lengths. This approach has been used in the Luminex xMAP systems (see, e.g., US Patent No. 6,939,720) and the Becton Dickinson Cytometric Bead Array systems, Alternatively, particles may be coded through differences in other physical properties such as size, shape, imbedded optical patterns and the like.

In certain embodiments of assays of the invention, particles may have a dual role as both i) a solid phase support used in an analyte concentration, collection and/or separation step and ii) as a detectable label or platform for detectable labels in a measurement step. In one example, a method of conducting a binding assay may comprise contacting a sample comprising an analyte with a particle linked to a first binding reagent that binds that analyte to form a complex comprising the analyte bound to the first binding reagent. The complex is then collected by collection of the particle (via magnetic collection, centrifugation, gravity sedimentation, etc.) and some or all of the unbound components of the sample are separated from the complex by removing some or all of the sample volume and, optionally, washing the collected particles. The complex is then released by resuspending the particles in the original or a new liquid media. The complex on the particle is then contacted with a second binding reagent bound to a solid phase, the second binding reagent binding the complex so as to bring the complex and particle to a surface of the solid phase. The amount of analyte in the sample is measured by measuring the amount of analyte bound to the solid phase, which in turn is measured by measuring the amount of particles bound to the solid phase (either by directly measuring the particles or by measuring detectable labels in or on the particles by, e.g., the measurement approaches described below).

The invention also includes assay methods that employ magnetic particles as detectable labels or as platforms for detectable labels in a binding assay. Advantageously, when using magnetic particles as a label or a label platform, a magnetic field can be applied to speed the kinetics for the binding of i) assay components linked to a magnetic particle to ii) binding reagents immobilized on a solid phase.

Accordingly, one embodiment is a method for conducting a binding assay comprising
(a) contacting (i) a sample comprising a target analyte with (ii) a magnetic particle linked to a first binding reagent that binds said target analyte and thereby forms a complex comprising said target analyte bound to said first binding reagent;
(b) contacting a solution comprising said complex with a second binding reagent bound to a solid phase, wherein said second binding reagent binds to said complex;
(c) applying a magnetic field to concentrate said particles near to said solid phase and thereby accelerating the rate of binding between said complex and said second binding reagent and
(d) measuring the amount of said analyte bound to said solid phase.

Optionally, such a method may also include, prior to step (b), collection and release steps as described elsewhere in this application so as to pre-concentrate said analyte and/or remove interferents from the sample. The magnetic particles used in such method are, preferably, between 10 nm and 10 um in diameter, more preferably between 50 nm and 1 um. The step of applying a magnetic field may be achieved through the use of permanent or electromagnets, e.g., by placing the magnet on the opposite side of the solid phase relative to the second binding reagent. Optionally, the magnet or magnetic field is translated and/or rotated along the solid phase so as to move the particles along the binding surface and allow the particles to interrogate the surface for available binding sites. Alternatively, or in conjunction with movement of the magnet/field, the magnetic field is intermittently removed and, while the magnetic field is removed, the particles are resuspended (e.g., by mixing) and then reconcentrated on the solid phase (thereby, allowing for allowing the particles to change rotational orientation on the surface and allowing them to interrogate additional areas on the surface. The method may also include a washing step, prior to the measuring step, to remove unbound particles. During such a washing step, the magnetic field is removed to allow for non-bound particles to be washed away. Alternatively, a magnetic field above the surface can be used to pull unbound particles away from the surface. The magnetic reaction acceleration approach may also be applied to multiplexed assay methods, as described elsewhere in this application, e.g., the solid phase may include an array of a plurality of different second binding reagents for use in array-based multiplexed measurements.

### (iv) Collection and Release

Collection, as used herein, refers to the physical localization of a material in a mixture. Collection includes the localization of a material through binding reactions or adsorption. For example, a material in a mixture may be collected on a solid phase by adsorption of the material on the solid phase or by binding of the material to binding reagents on the solid phase. Collection is not, however, limited to localization at a solid phase and may also include techniques in the art for localizing materials at a location/volume within a larger fluid volume, for example, localization of materials through the use of optical tweezers (which use light to manipulate microscopic objects as small as a single atom, wherein the radiation pressure from a focused laser beam is able to trap small particles), electric or magnetic fields, focused flow, density gradient centrifugation, etc.

Certain embodiments of the invention include the collection of microparticles or materials that are bound to microparticles. Suitable collection methods include the many methods known in the art of microparticle-based assays that achieve localization of microparticles from a suspension. These include sedimentation under gravity or by centrifugation, filtration onto a filter or porous membrane, localization (of magnetizable particles) by application of a magnetic field, binding or adsorption of the particles to a macroscopic solid phase, use of optical tweezers, etc.

Release, as used herein, refers to delocalization of a previously collected material. Materials that are held at a localized position through chemical bonds or through specific or non-specific binding interactions may be allowed to delocalize by breaking the bond or interaction so that the materials may diffuse or mix into the surrounding media. There are many well-established cleavable chemical linkers that may be used that provide a covalent bond that may be cleaved without requiring harsh conditions. For example, disulfide containing linkers may be cleaved using thiols or other reducing agents, cis-diol containing linkers may be cleaved using periodate, metal-ligand interactions (such as nickel-histidine) may be cleaved by changing pH or introducing competing ligands. Similarly, there are many well-established reversible binding pairs that may be employed (including those that have been identified in the art of affinity chromatography). By way of example, the binding of many antibody-ligand pairs can be reversed through changes in pH, addition of protein denaturants or chaotropic agents, addition of competing ligands, etc. Other suitable reversible binding pairs include complementary nucleic acid sequences, the hybridization of which may be reversed under a variety of conditions including changing pH, increasing salt concentration, increasing temperature above the melting temperature for the pair and/or adding nucleic acid denaturants (such as formamide). Such reversible binding pairs may be used as targeting agents (as described above), e.g., a first targeting agent may be linked to a first binding reagent that binds an analyte, a second targeting agent may be linked to a solid phase, and a binding interaction of the first and second targeting agents may be used to reversibly immobilize the first binding reagent on the solid phase.

Release also includes physical delocalization of materials by, for example, mixing, shaking, vortexing, convective fluid flow, mixing by application of magnetic, electrical or optical forces and the like. Where microparticles or materials bound to microparticles have been collected, such physical methods may be used to resuspend the particles in a surrounding matrix. Release may simply be the reverse of a previous collection step (e.g., by any of the mechanisms described above) or collection and release could proceed by two different mechanisms. In one such example, collection of materials (such as an analyte or a complex comprising an analyte) bound to a particle can be achieved by physical collection of the particle. The materials are then released by cleaving a bond or reversing a binding reaction holding the material on the particle. In a second such example, materials (such as an analyte of a complex comprising an analyte are collected on a surface through a binding interaction with a binding reagent that is linked to the surface. The material is then released by breaking a bond or a second binding interaction linking the binding reagent to the surface.

Collection followed by release may be used to concentrate and/or purify analytes in a sample. By collecting in a first volume and releasing into a second smaller volume, an analyte in a sample may be concentrated. Through concentration, it is often possible to significantly improve the sensitivity of a subsequent measurement step. By collecting from a sample and removing some or all of the uncollected sample, potential assay interferents in the sample may be reduced or eliminated. Optionally, removal of the unbound sample may include washing a collected material with and releasing the collected material into defined liquid reagents (e.g., assay or wash buffers) so as to provide a uniform matrix for subsequent assay steps.

### (iv) Measurement Methods

The methods of the invention can be used with a variety of methods for measuring the amount of an analyte and, in particular, measuring the amount of an analyte bound to a solid phase. Techniques that may be used include, but are not limited to, techniques known in the art such as cell culture-based assays, binding assays (including agglutination tests, immunoassays, nucleic acid hybridization assays, etc.), enzymatic assays, colorometric assays, etc. Other suitable techniques will be readily apparent to one of average skill in the art. Some measurement techniques allow for measurements to be made by visual inspection, others may require or benefit from the use of an instrument to conduct the measurement.

Methods for measuring the amount of an analyte include label free techniques, which include but are not limited to i) techniques that measure changes in mass or refractive index at a surface after binding of an analyte to a surface (e.g., surface acoustic wave techniques, surface plasmon resonance sensors, ellipsometric techniques, etc.), ii) mass spectrometric techniques (including techniques like MALDI, SELDI, etc. that can measure analytes on a surface), iii) chromatographic or electrophoretic techniques, iv) fluorescence techniques (which may be based on the inherent fluorescence of an analyte), etc.

Methods for measuring the amount of an analyte also include techniques that measure analytes through the detection of labels which may be attached directly or indirectly (e.g., through the use of labeled binding partners of an analyte) to an analyte. Suitable labels include labels that can be directly visualized (e.g., particles that may be seen visually and labels that generate an measurable signal such as light scattering, optical absorbance, fluorescence, chemiluminescence, electrochemiluminescence, radioactivity, magnetic fields, etc). Labels that may be used also include enzymes or other chemically reactive species that have a chemical activity that leads to a measurable signal such as light scattering, absorbance, fluorescence, etc. The use of enzymes as labels has been well established in in Enzyme-Linked ImmunoSorbent Assays, also called ELISAs, Enzyme ImmunoAssays or EIAs. In the ELISA format, an unknown amount of antigen is affixed to a surface and then a specific antibody is washed over the surface so that it can bind to the antigen. This antibody is linked to an enzyme, and in the final step a substance is added that the enzyme converts to a product that provides a change in a detectable signal. The formation of product may be detectable, e.g., due a difference, relative to the substrate, in a measurable property such as absorbance, fluorescence, chemiluminescence, light scattering, etc. Certain (but not all) measurement methods that may be used with solid phase binding methods according to the invention may benefit from or require a wash step to remove unbound components (e.g., labels) from the solid phase Accordingly, the methods of the invention may comprise such a wash step.

In one embodiment, an analyte(s) of interest in the sample may be measured using electrochemiluminescence-based assay formats, e.g. electrochemiluminescence (ECL) based immunoassays. The high sensitivity, broad dynamic range and selectivity of ECL are important factors for medical diagnostics. Commercially available ECL instruments have demonstrated exceptional performance and they have become widely used for reasons including their excellent sensitivity, dynamic range, precision, and tolerance of complex sample matrices. Species that can be induced to emit ECL (ECL-active species) have been used as ECL labels, e.g., i) organometallic compounds where the metal is from, for example, the noble metals of group VIII, including Ru-containing and Os-containing organometallic compounds such as the tris-bipyridyl-ruthenium (RuBpy) moiety and ii) luminol and related compounds. Species that participate with the ECL label in the ECL process are referred to herein as ECL coreactants. Commonly used coreactants include tertiary amines (e.g., see U.S. Patent No. 5,846,485), oxalate, and persulfate for ECL from RuBpy and hydrogen peroxide for ECL from luminol (see, e.g., U.S. Patent No. 5,240,863). The light generated by ECL labels can be used as a reporter signal in diagnostic procedures (Bard et al., U.S. Patent No. 5,238,808, herein incorporated by reference). For instance, an ECL label can be covalently coupled to a binding agent such as an antibody, nucleic acid probe, receptor or ligand; the participation of the binding reagent in a binding interaction can be monitored by measuring ECL emitted from the ECL label. Alternatively, the ECL signal from an ECL-active compound may be indicative of the chemical environment (see, e.g., U.S. Patent No. 5,641,623 which describes ECL assays that monitor the formation or destruction of ECL coreactants). For more background on ECL, ECL labels, ECL assays and instrumentation for conducting ECL assays see U.S. Patents Nos. 5,093,268; 5,147,806; 5,324,457; 5,591,581; 5,597,910; 5,641,623; 5,643,713; 5,679,519; 5,705,402; 5,846,485; 5,866,434; 5,786,141; 5,731,147; 6,066,448; 6,136,268; 5,776,672; 5,308,754; 5,240,863; 6,207,369; 6,214,552 and 5,589,136 and Published PCT Nos. WO99/63347; WOOO/03233; WO99/58962; WO99/32662; WO99/14599; WO98/12539; WO97/36931 and WO98/57154, all of which are incorporated herein by reference.

The capture/collection and release methods of the invention may be applied to singleplex or multiplex formats where multiple assay measurements are performed on a single sample. Multiplex measurements that can be used with the invention include, but are not limited to, multiplex measurements i) that involve the use of multiple sensors; ii) that use discrete assay domains on a surface (e.g., an array) that are distinguishable based on location on the surface; iii) that involve the use of reagents coated on particles that are distinguishable based on a particle property such as size, shape, color, etc.; iv) that produce assay signals that are distinguishable based on optical properties (e.g., absorbance or emission spectrum) or v) that are based on temporal properties of assay signal (e.g., time, frequency or phase of a signal).

### (v) Assay Formats

One embodiment of the present invention employs a specific binding assay, e.g., an immunoassay, immunochromatographic assay or other assay that uses a binding reagent. The immunoassay or specific binding assay according to one embodiment of the invention can involve a number of formats available in the art. The antibodies and/or specific binding partners can be labeled with a label or immobilized on a surface. Thus, in one embodiment, the detection method is a binding assay, e.g., an immunoassay, receptor-ligand binding assay or hybridization assay, and the detection is performed by contacting an assay composition with one or more detection molecules capable of specifically binding with an analyte(s) of interest in the sample.

In one embodiment, the assay uses a direct binding assay format. An analyte is bound to a binding partner of the analyte, which may be immobilized on a solid phase. The bound analyte is measured by direct detection of the analyte or through a label attached to the analyte (e.g., by the measurements described above).

In one embodiment, the assay uses a sandwich or competitive binding assay format. Examples of sandwich immunoassays performed on test strips are described in U.S. Pat. No. 4,168,146 to Grubb et al. and U.S. Pat. No. 4,366,241 to Tom et al., both of which are incorporated herein by reference. Examples of competitive immunoassay devices suitable for use with the present methods include those disclosed in U.S. Pat. No. 4,235,601 to Deutsch et al., U.S. Pat. No. 4,442,204 to Liotta, and U.S. Pat. No. 5,208,535 to Buechler et al., all of which are incorporated herein by reference.

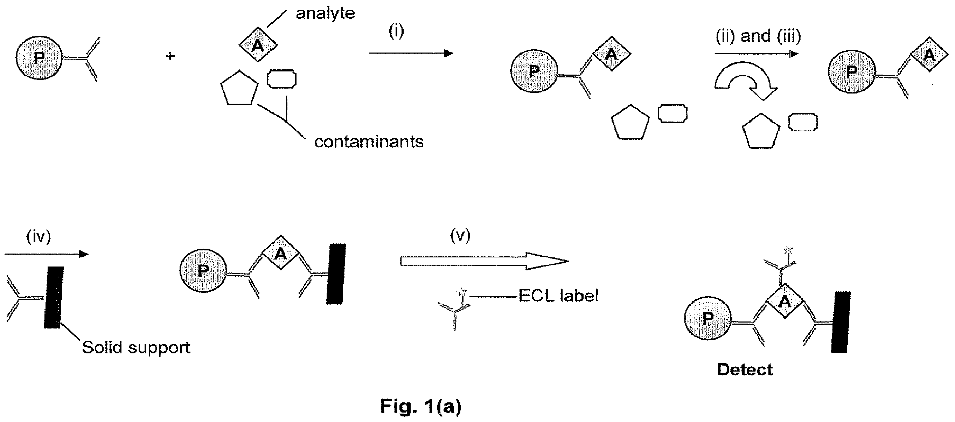

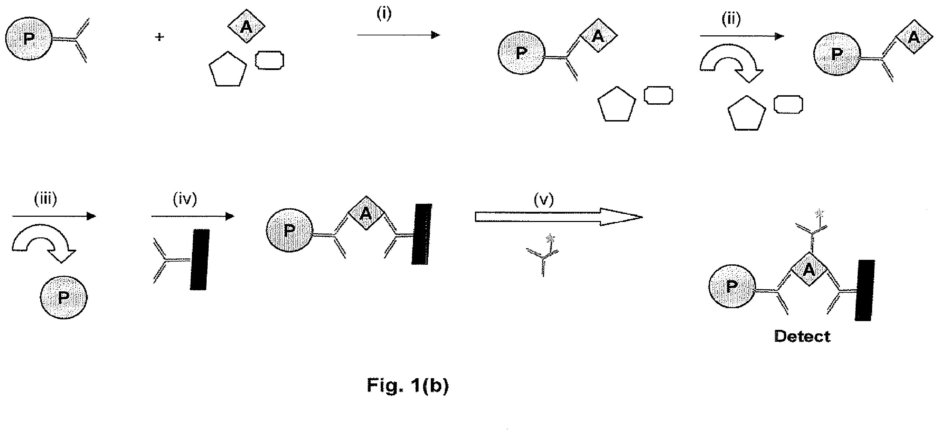

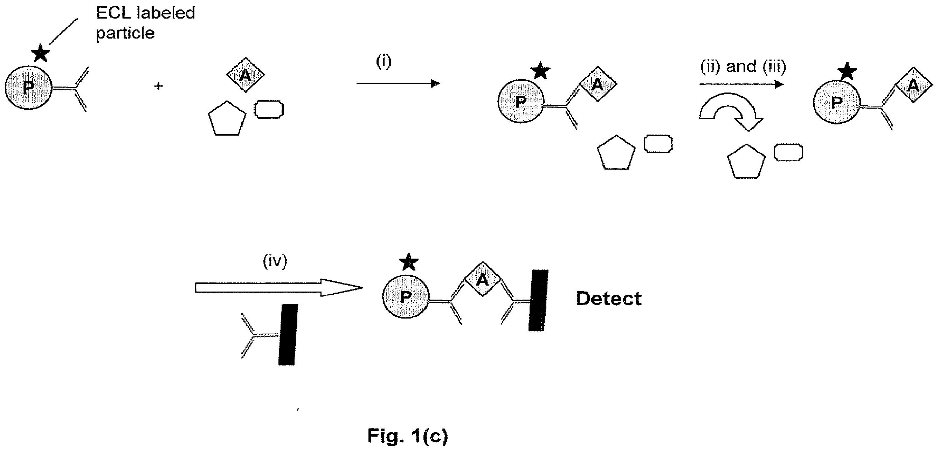

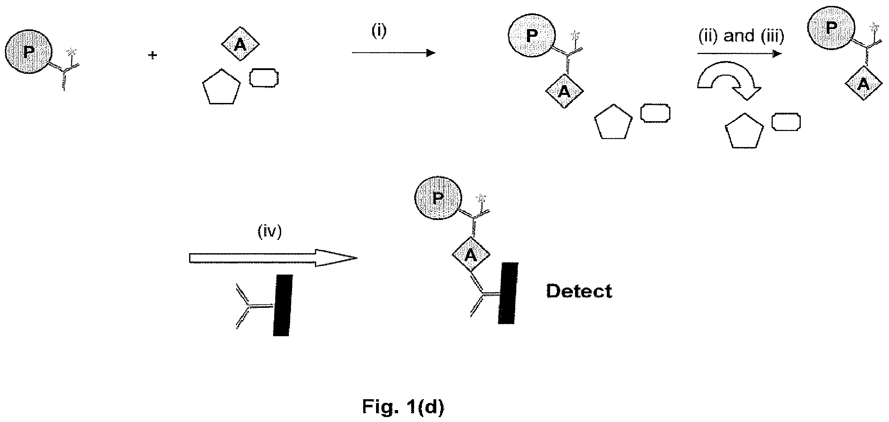

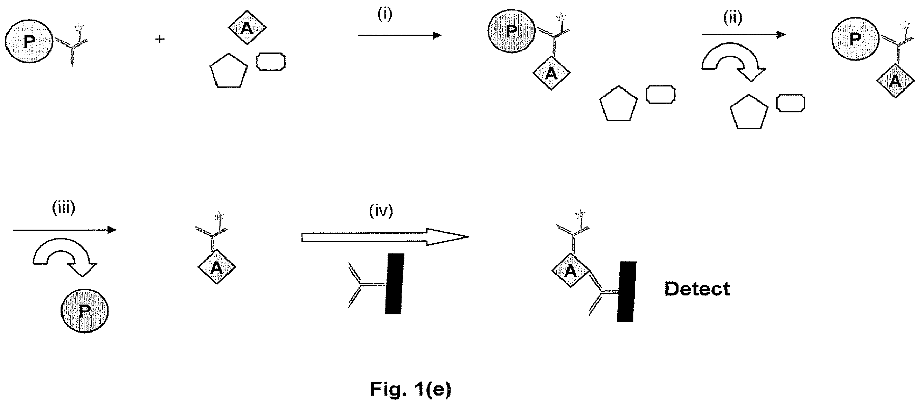

In a sandwich assay, analyte in the sample is bound to a first binding reagent and a second labeled binding reagent and the formation of this "sandwich" complex is measured. In a solid phase sandwich assay, the first binding reagent is immobilized on a solid phase and the amount of labeled antibody on the solid phase, due to formation of the sandwich complex, is then measured. The signal generated in a sandwich assay will generally have a positive correlation with the concentration of the analyte. Various configurations of sandwich assays that use the methods of the present invention are shown in Figs. 1-4. In one embodiment, e.g., in Fig. 1(a), the assay includes contacting a sample comprising a target analyte with a particle or solid phase linked to a first binding reagent that binds the target analyte, thereby forming a complex comprising the target analyte bound to the first binding reagent. The complex is collected, separated and released, as described herein, and then a sandwich is formed by contacting the complex with an additional binding reagent (e.g., a second binding reagent). As shown in Fig. 1(a) and Fig. 1(b), the particle or solid phase may or may not be cleaved from the complex prior to contacting the complex with an additional binding reagent.

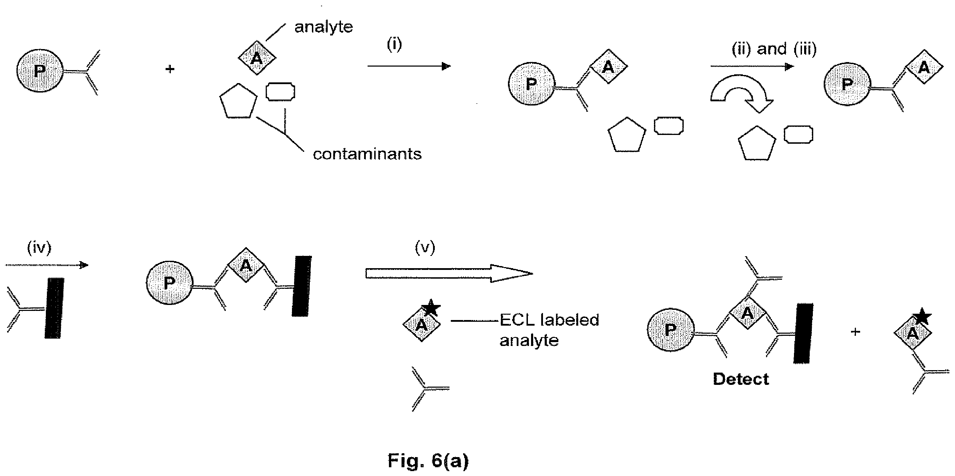

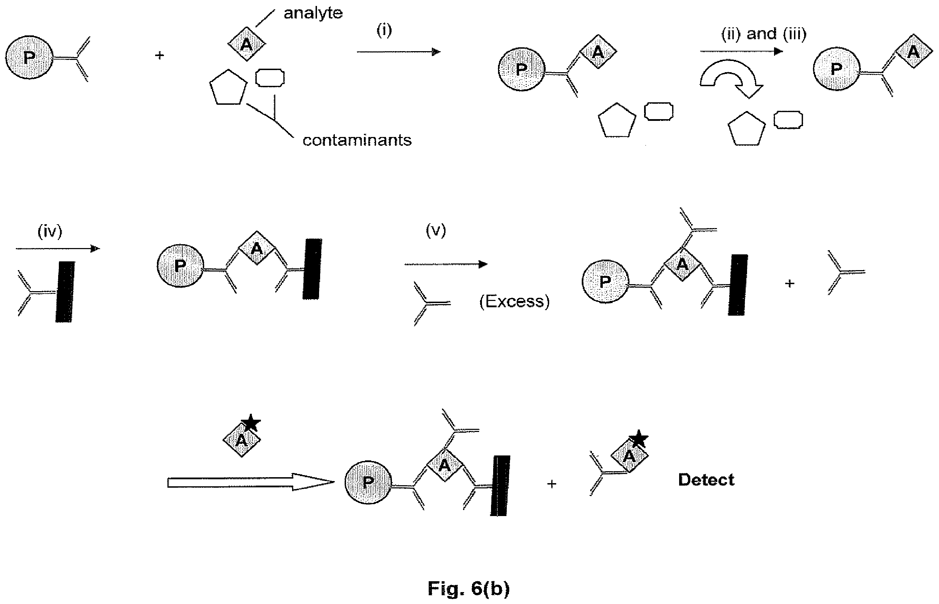

In a competitive assay, unlabelled analyte in the test sample is measured by its ability to compete with labeled or immobilized analyte. In the example of competitive assays employing labeled analytes, the unlabeled analyte in a sample blocks the ability of the labeled analyte to bind a binding reagent by occupying the binding site. Thus, in a competitive assay, the signal generated has an inverse correlation with the concentration of analyte in a sample. Figs. 6(a) and 6(b) show the use of the methods of the present invention in a two step competitive format. As in Fig. 1(a), the analyte of interest in the sample is pre-concentrated. Labeled analyte bound to a solid support is incubated with the pre-concentrated analyte complex. Figs. 6(a) and 6(b) serve to illustrate how the methods of the present invention may be used in a competitive assay format. The skilled artisan will understand that alternate configurations of a competitive immunoassay may be achieved using the methods of the present invention without undue experimentation.

### (vi) Specific Embodiments

In one embodiment, a method is provided for conducting a binding assay comprising contacting a sample comprising a target analyte, A, and which may also contain various sample contaminants as shown in Fig. 1(a), with a particle linked to a first binding reagent that binds the target analyte and thereby forms a complex comprising the target analyte bound to the first binding reagent. Once the sample is mixed with the particle to form the complex, the complex is collected. This collection step may involve accumulation of the complex at a surface, e.g., by centrifugation of the particles, allowing the particles to rise or settle under gravity, filtering the particles onto a filtration media, magnetically collecting the particles (in the case of magnetic particles), etc. Alternatively, the collection step may involve accumulation of the complex within a defined volume within the sample, e.g., by holding the particles in this defined volume through the use of optical tweezers or focused flow. Optionally, the unbound components of the sample are then separated from the complex, e.g., by removing all or part of the non-collected components and/or by washing the collected complex with an additional assay medium or wash buffer. Thereafter, the complex is released, e.g., resuspended into the assay medium, and the complex is contacted with a second binding reagent bound to a solid phase, wherein the second binding reagent binds to the complex. The amount of analyte is detected by measuring the amount of a detectable label linked to an assay component bound to the solid phase. The detectable label may be linked to the first binding reagent, an optional third binding reagent, if one is used in the assay format, the particle or an additional assay component that is comprised within or bound to the complex.

A variety of approaches are provided for conducting the collection and release steps described above and for providing the labeled reagent. Fig 1(a) shows a method with the following steps: (i) a first binding reagent linked to a particle binds to the analyte to form a complex, (ii and iii) the complex is collected and released by collection and resuspension of the particle during which steps the analyte may be concentrated and/or separated from contaminants in the sample, (iv) the complex binds to a second binding reagent on a solid phase and (v) the complex is contacted with a labeled third binding reagent that binds the analyte in the complex such that it can be detected. Fig 1(b) shows a method similar to the one in Fig 1(a), except that the complex is released in step (iii) by cleaving the first binding reagent from the particle instead of simply resuspending the particle. Figs 1(c) and 1(d) show methods similar to the one in Fig 1(a) except that that the label is attached to (or incorporated within) the particle (Fig 1(c)) or attached to the first binding reagent (Fig 1(d)) and the step of contacted the complex with a labeled third binding reagent is omitted. Alternatively, if the particle is measured directly (e.g., by direct visual observation of the particle), the label may be omitted. Fig 1(e) shows a method similar to the one in Fig 1(b) except that the label is attached to the first binding reagent and the step of contacting the complex with a labeled third binding reagent is omitted.

The measuring step may comprise any suitable method of measuring the presence of a detectable label in a sample (see the Measurement Methods section), e.g., optical absorbance, fluorescence, phosphorescence, chemiluminescence, light scattering or magnetism. In one embodiment, the detectable label is an electrochemiluminescent label and the measuring step comprises measuring an ECL signal and correlating that signal with an amount of analyte in the sample. Thus, the measuring step may further comprise contacting the complex with an electrode and applying a voltage waveform to the electrode to generate ECL.

The methods described in Figs 1(a)-1(e) may be applied to multiplex measurements for multiple analytes in a sample. In such methods, the first, second and third binding reagents (if present) may be selected to bind multiple analytes (e.g., the use of poly-dT as a binding reagent to capture multiple mRNAs in a sample through the common poly-dA tail sequence) or, alternatively; the methods may employ a plurality of different first binding reagents, second binding reagents and/or third binding reagents to bind to the multiple analytes. To allow for independent measurement of different analytes, such multiplex methods employs at least one of the group consisting of i) a plurality of different first binding reagents, ii) a plurality of second binding reagents and iii) a plurality of third binding reagents (the different reagents within (i), (ii) or (iii) being selected for their ability to preferentially bind a target analyte relative to other target analytes). Where a plurality of first binding reagents are used, individual particles may be attached to mixtures of the different first binding reagents or, alternatively, the particles may be prepared so that individual particles are attached to only one type of first binding reagent (e.g., such that an individual particle preferentially binds one of the target analytes relative to other target analytes).

The multiplex methods may use a variety of approaches for independently measuring different analytes. In one embodiment, a plurality of labeled binding reagents with different preferences for target analytes may be used (e.g., a plurality of different labeled third binding reagents as in Figs 1(a) and 1(b), a plurality of different labeled first binding reagents as in Fig 1(e) or a plurality of different labeled first binding reagent-particle conjugates as in Figs 1(c) and 1(d)). The labels on the different labeled reagents (or, alternatively, the particles in the particle conjugates) are selected to provide distinguishable assay signals such that the different labeled reagents and, therefore, the different target analytes, can be measured independently. In another embodiment, a plurality of second binding reagents with different preferences for target analytes may be used. The different second binding reagents may be patterned into different discrete binding domains on one or more solid phases (e.g., as in a binding array) such that assay signals generated on the different binding domains and, therefore, the different analytes, can be measured independently (e.g., by independently addressing binding domains on electrode arrays or by independently measuring light emitted from different binding domains in a luminescence assay). Alternatively, the different second binding reagents may be coupled to different coded beads (as described in the Solid Phases section) to allow for the different analytes to be measured independently.

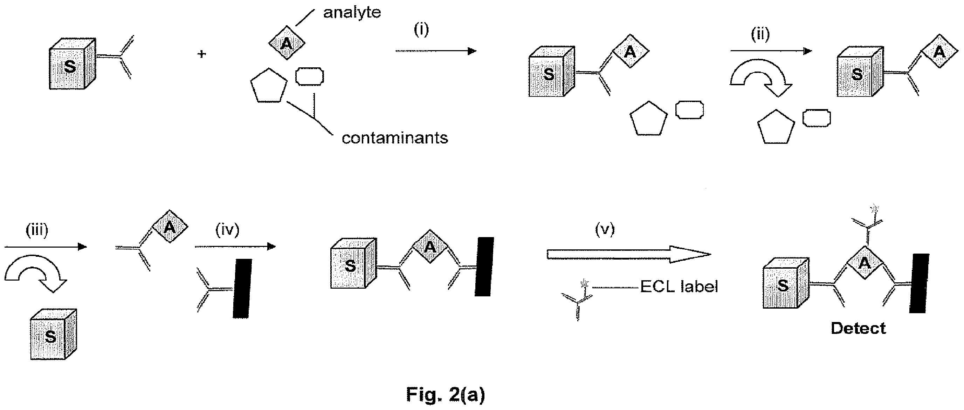

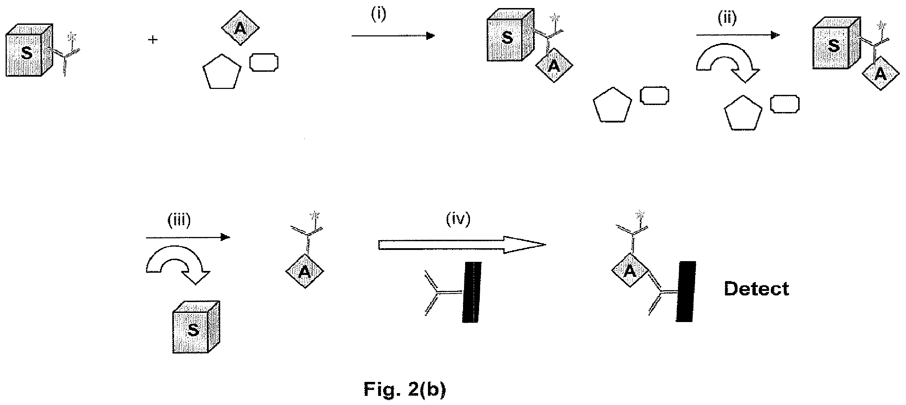

In an alternative embodiment, a method of conducting a binding assay is provided as shown in Figs. 2(a)-2(b), which comprises contacting a sample comprising a target analyte with a first solid phase, S, linked to a first binding reagent that binds the target analyte and forms a complex comprising the target analyte bound to the first binding reagent. Once the sample is contacted with the first solid phase, the unbound components of the sample are separated from the complex, the complex is released from the solid phase into the assay medium and the first solid phase is removed from the first binding reagent. Thereafter, the released complex is contacted with a second solid phase comprising a second binding reagent that binds to the complex, and the amount of analyte bound to the second solid phase is quantified. The detectable label may be linked to the first binding reagent, an optional third binding reagent, if one is used in the assay format, the particle or an additional assay component that is comprised within or bound to the complex. In Fig. 2(a), the label is attached to a third binding reagent (and the method includes the step of contacting the complex with the third binding reagent), whereas the label is attached to the first binding reagent in Fig. 2(b).

As described for Fig 1, the methods described in Fig 2 may also be extended to multiplex measurements, e.g., by employing at least one of the group consisting of i) a plurality of different first binding reagents, ii) a plurality of second binding reagents and iii) a plurality of third binding reagents (the different reagents within (i), (ii) or (iii) being selected for their ability to preferentially bind a target analyte relative to other target analytes).

The invention also provides a method of conducting a multiplexed binding assay for a plurality of analytes that includes contacting (i) a sample with (ii) one or more first solid phases linked to one or more first binding reagents that bind the analytes to form complexes comprising the analytes bound to the first binding reagents. The unbound components of the sample are, optionally, separated from the complexes. The complexes are released and then contacted with a plurality of binding domains comprising second binding reagents that bind to said complexes, wherein each binding domain comprises a second binding reagent that binds to a complex comprising a secondary target analyte. Thereafter, the amount of analyte bound to the binding domains is measured.

According to another embodiment, a multiplexed assay may comprise the acts of contacting at least a portion of a sample with one or more binding surfaces comprising a plurality of binding domains, immobilizing one or more analytes on the domains and measuring the analytes immobilized on the domains. In certain embodiments, at least two of the binding domains differ in their specificity for analytes of interest. In one example of such an embodiment, the binding domains are prepared by immobilizing, on one or more surfaces, discrete domains of binding reagents that bind analytes of interest. Optionally, the sample is exposed to a binding surface that comprises an array of binding reagents. Optionally, the surface(s) may define, in part, one or more boundaries of a container (e.g., a flow cell, well, cuvette, etc.) which holds the sample or through which the sample is passed. The method may also comprise generating assay signals that are indicative of the amount of the analytes in the different binding domains, e.g., changes in optical absorbance, changes in fluorescence, the generation of chemiluminescence or electrochemiluminescence, changes in reflectivity, refractive index or light scattering, the accumulation or release of detectable labels from the domains, oxidation or reduction or redox species, electrical currents or potentials, changes in magnetic fields, etc.

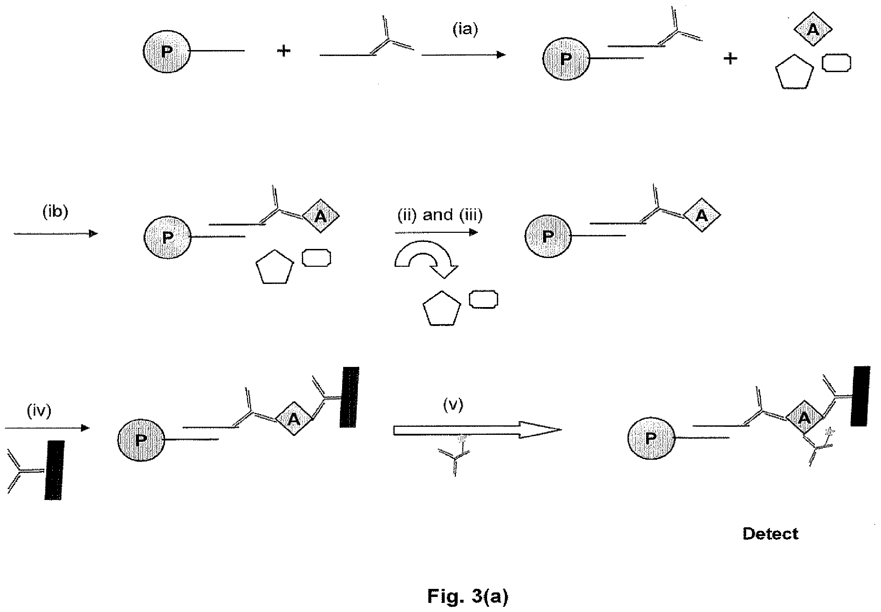

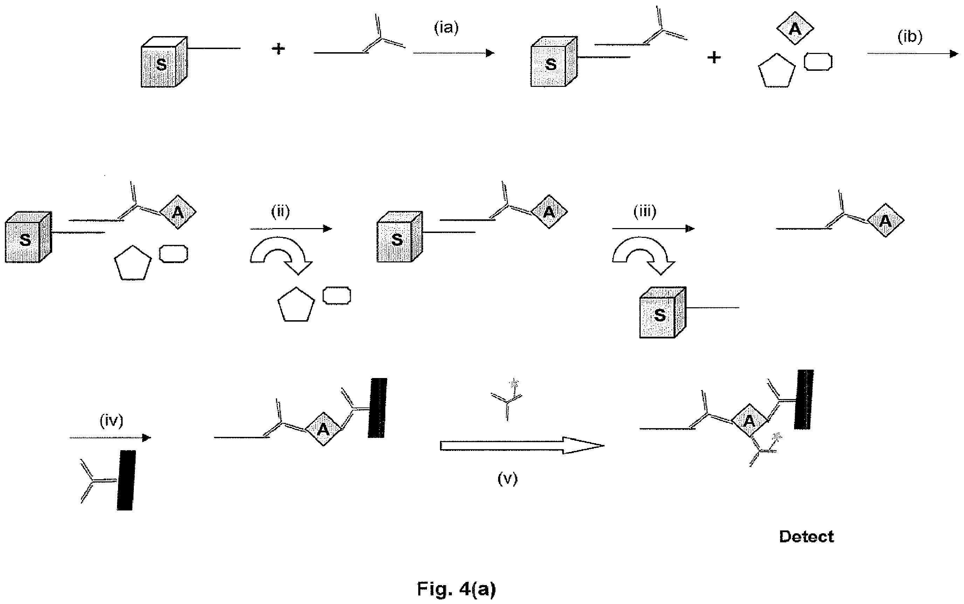

Assays of certain embodiments of the invention may employ targeting agents to link the target analyte with a binding reagent in the assay medium. Such assay formats are illustrated in Figs. 3(a)-3(e) and Figs. 4(a)-4(b), which are analogous to Figs 1(a)-1(e) and Figs 2(a)-2(b), except that the binding of analyte to a first binding reagent on a solid phase/particle takes place through two steps: (i(a)) contacting the first binding reagent linked to a first targeting agent to a particle (or other solid phase) linked to a second targeting agent that binds to said first targeting agent (thus attaching said first binding reagent to the particle or other solid phase) and (i(b)) contacting said first binding reagent with a sample comprising a target analyte that binds said first binding reagent. Step i(a) may occur before step i(b) (as shown in the figures) or the two steps may occur in the reverse order or concurrently. Steps i(a) and i(b) may both be carried out during the conduct of an assay or, alternatively, the first binding reagent may be supplied to the user pre-bound to the solid phase through the targeting agents (e.g., if the targeting agents were pre-bound during manufacturing), in which case step i(a) may be omitted.

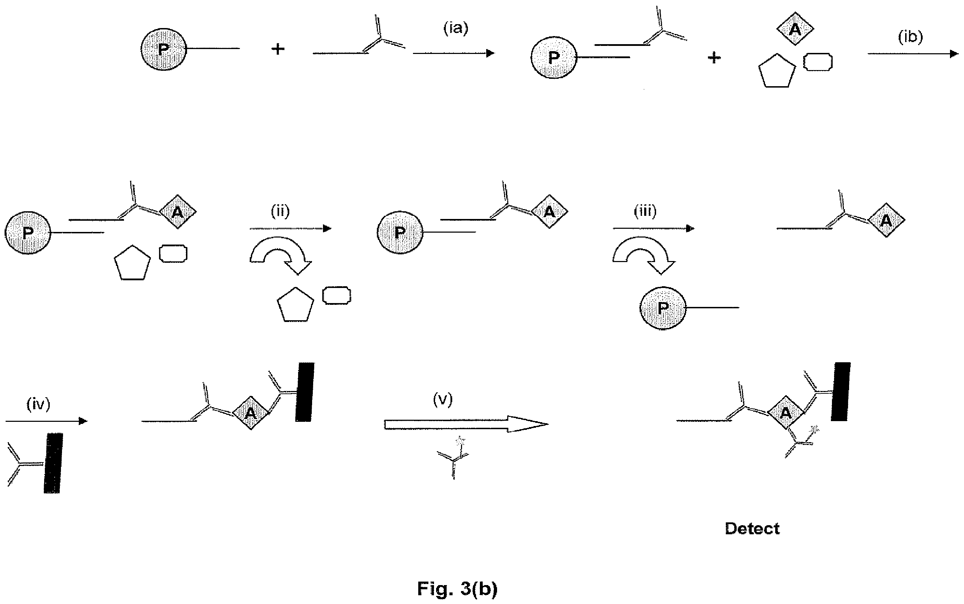

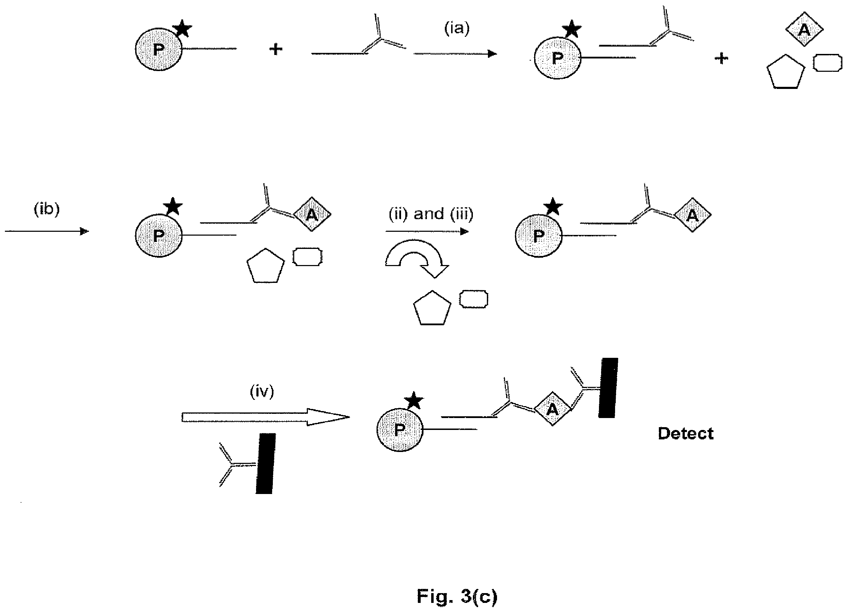

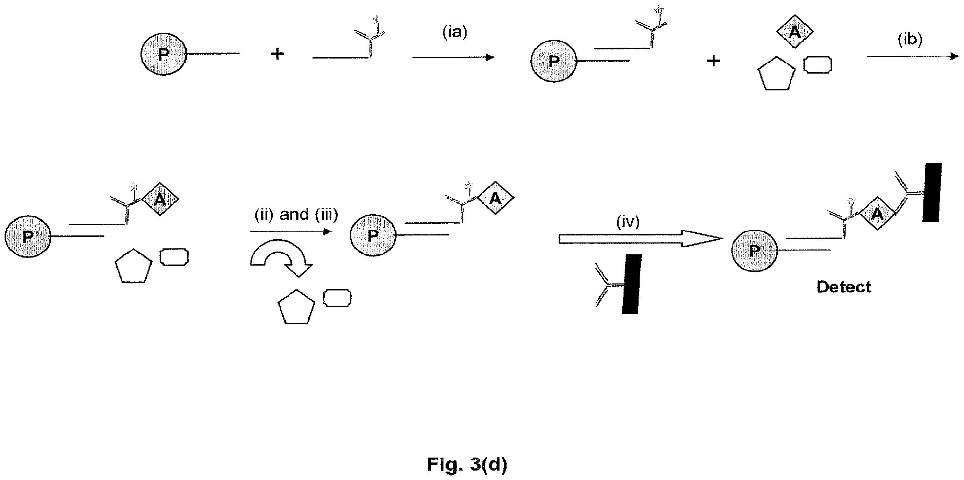

Thus, in one embodiment, the method includes contacting a sample comprising a target analyte with a particle linked to a first binding reagent that binds the target analyte, wherein the first binding reagent is linked to a first targeting agent and the particle is linked to a second targeting agent, and the first binding reagent and the particle are linked via a binding reaction between the first and second targeting agents to form a complex comprising said target analyte bound to said first binding reagent (see e.g., Fig. 3(a)). The complex is then collected and unbound components in the sample are separated from the complex. The complex is released and the released complex is contacted with a second binding reagent bound to a solid phase, wherein the second binding reagent binds to the complex. The amount of analyte bound to the solid phase is measured. As in the embodiments described above and illustrated in Figs. 1(a)-1(e), the detectable label may be attached to various assay components in the medium, e.g., to a third binding reagent, as in Figs. 3(a)-3(b), to the particle, as in Fig. 3(c), or to the first binding reagent, as in Figs. 3(d)-3(e). Moreover, the complex is optionally cleaved from the particle prior to the detection step, as in Figs. 3(b) and 3(d).

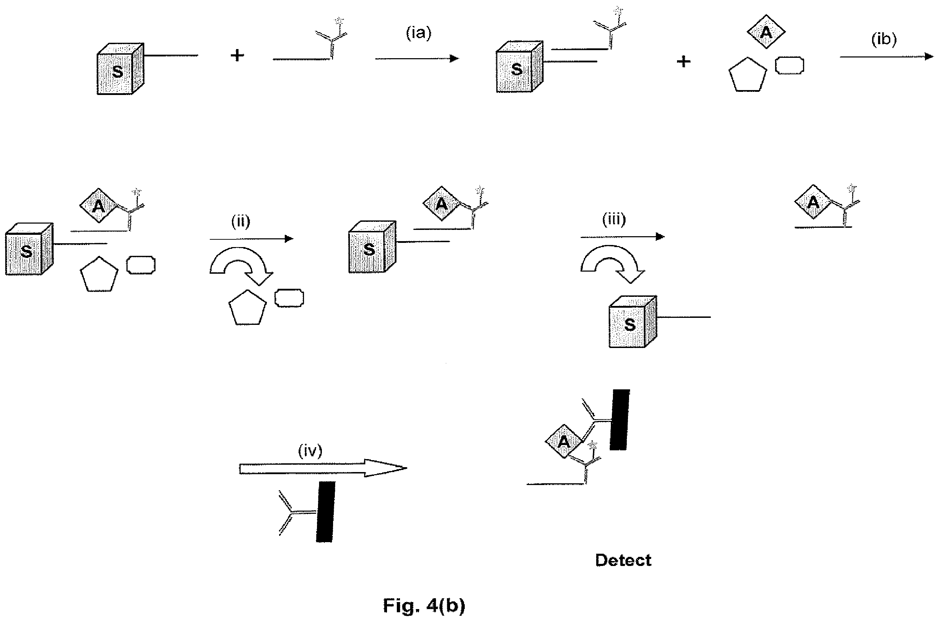

In one embodiment, the assay may include (a) contacting a sample comprising a target analyte with a first solid phase linked to a first binding reagent that binds the target analyte, wherein the first binding reagent is linked to a first targeting agent and the first solid phase is linked to a second targeting agent, and the first binding reagent and the first solid phase are linked via a binding reaction between the first and second targeting agents to form a complex comprising said target analyte bound to said first binding reagent (see e.g., Figs. 4(a)-4(b)). The complex is then collected and unbound components in the sample are separated from the complex. The complex is released, e.g., resolubilized, and the first solid phase is removed. The released complex is contacted with a second binding reagent bound to a second solid phase, wherein the second binding reagent binds to the complex. The amount of analyte bound to the second solid phase is measured. The detectable label may be attached to any suitable assay component, e.g., the first binding reagent, as in Fig. 4(b), or the third binding reagent, as in Fig. 4(a).

The releasing step in the various assay formats described herein may comprise cleaving a binding reagent from the particle (e.g., as shown in Fig. 1(b)). This may be accomplished by any suitable method, e.g., subjecting the complex to increased temperature, pH changes, altering the ionic strength of the solution, competition, and combinations thereof.

If a targeting agent is employed in the assay format, the releasing step comprises disassociating the first and second targeting agents, e.g., by subjecting the complex to increased temperature, pH changes, altering the ionic strength of the solution, competition, and combinations thereof as discussed above.

The measuring step in the various assay formats described herein may comprise any suitable method of measuring the presence of a detectable label in a sample, e.g., optical absorbance, fluorescence, phosphorescence, chemiluminescence, light scattering or magnetism. In one embodiment, the detectable label is an electrochemiluminescent label and the measuring step comprises measuring an ECL signal and correlating that signal with an amount of analyte in the sample. Thus, the measuring step may further comprise contacting the complex with an electrode and applying a voltage waveform to the electrode to generate ECL.

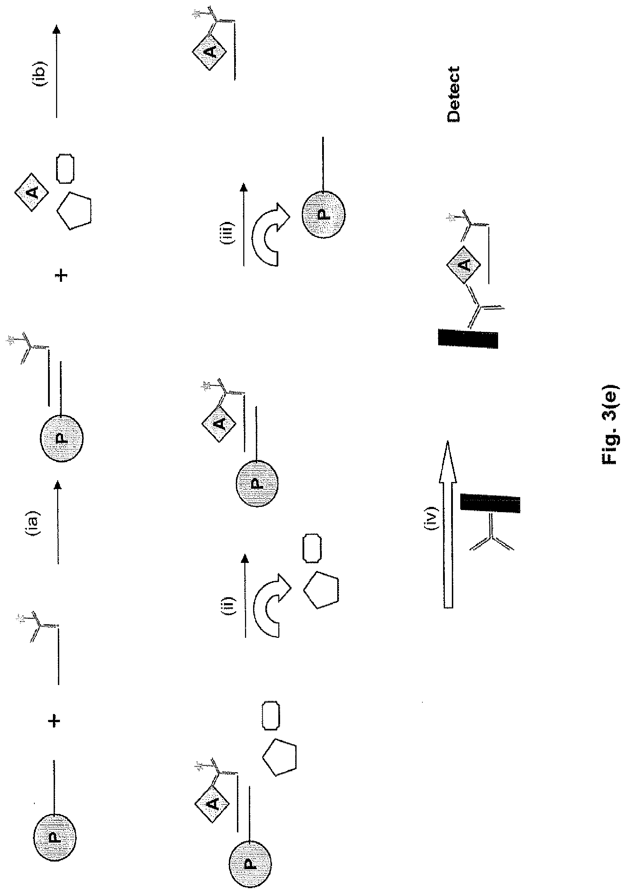

By analogy to the description of Figures 1 and 2, the methods in Figures 3 and 4 may also be extended to multiplex measurements, e.g., by employing at least one of the group consisting of i) a plurality of different first binding reagents, ii) a plurality of second binding reagents and iii) a plurality of third binding reagents (the different reagents within (i), (ii) or (iii) being selected for their ability to preferentially bind a target analyte relative to other target analytes). In such multiplex methods, a common targeting reagent pair may be used to link a plurality of different first binding reagents to the corresponding particles or other solid phases. Alternatively, a unique targeting reagent pair may be used for each different first binding reagent (e.g., a different set of complementary oligonucleotides may be used to target each of the different first binding reagents). Such an approach may be used to i) target different first binding reagents to different distinguishable particles (e.g., particles bearing distinguishable labels) or ii) enable multiplexing through the use of a plurality of different second binding reagents, each of which binds preferentially to a different first targeting agent (thus preferentially binding complexes comprising one of the plurality of analytes).

### EXAMPLES

### Example 1 - Dual use of labeled magnetic particle to concentrate and detect analytes of interest

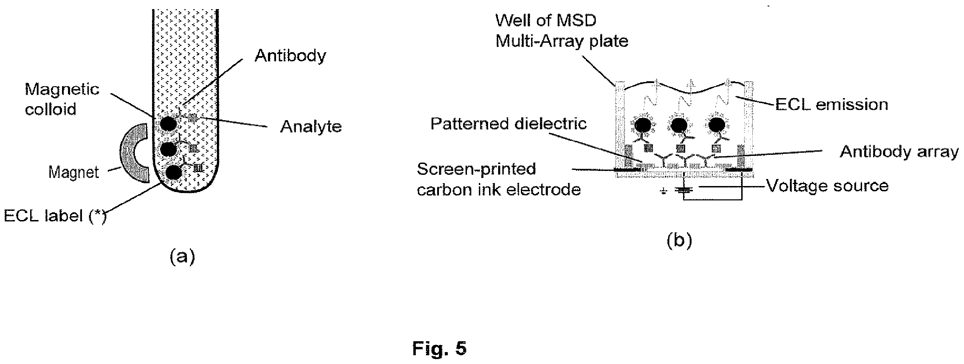

As shown in Figure 5, magnetic particles are coated with antibodies against the analytes of interest and a large number (e.g., greater than 100) ECL labels, By attachment of the ECL labels to the antibodies (either before or after coating the antibodies on the particles), very high numbers of labels can be easily achieved. A particle of only 60 nm in diameter can support roughly 160 antibody molecules, assuming about 50 nm² of surface area per antibody. Thus, attachment of only 1 label per antibody allows labeling ratios of greater than 100 labels per particle to be achieved for 60 nm particles. Labeling ratios of greater than 1000 labels per particle are achieved by increasing the number of labels per antibody and/or increasing the particle size).

A 1 mL or greater volume of sample is combined with the particles in a container and after incubating the mixture to allow the antibodies to bind their respective targets, a magnetic field is applied such that the magnetic particles collect on a surface in the container (a variety of commercial magnetic tube holders or probes are available for carrying out this step). The complexes are washed with buffered saline to remove unbound components of the sample. The magnetic field is removed and the particles are then re-suspended in 100 uL of a suitable assay diluent, thus providing a 10-fold or greater increase in concentration relative to the original sample. The particle-analyte complexes are transferred to an assay plate (e.g., a MULTI-ARRAY® 96-well assay plate, Meso Scale Diagnostics, LLC, Gaithersburg, MD) that includes a binding surface comprising an array of antibody binding reagents directed against the analytes of interest. Complexes that bind the array are measured by ECL on a SECTOR® Imager instrument (Meso Scale Diagnostics, LLC). The magnetic collection step provides for improvements in assay performance by allowing for pre-concentration of analyte into a small volume and removal of potential interferents in the sample.

### Example 2 - Assay using antibodies coupled to magnetic particles through oligonucleotide hybridization reactions

Magnetic particles are coated with oligonucleotides and a large number (greater than 100) ECL labels. Conjugates are formed comprising antibodies against analytes of interest and oligonucleotides complementary to the oligonucleotides on the particles. The antibody conjugates and particles are subjected to conditions sufficient to hybridize the complementary oligonucleotide sequences (e.g., appropriate temperature, ionic strength and denaturing conditions, as described hereinabove) and thereby coat the antibodies on the particles. These particles are then used to assay for analytes of interest as described in Example 1.

### Example 3 - Demonstration of the release of antibodies coupled to magnetic particles through oligonucleotide hybridization reactions

Magnetic beads (Dynalbeads® MyOne™-Streptavidin Cl beads, Invitrogen Corporation) were coated with a biotinylated oligonucleotide by the following procedure: The beads (3 mg) were washed three times at 60°C in hybridization buffer (20 mM Tris, 1 mM EDTA, 250 mM NaCl, 0.01% Triton-X at pH=8 and 0.1% BSA). The beads were then coated at room temperature with 750 pmoles of a 19-mer biotinylated oligonucleotide (Oligo 1, Tm = 40°C), in 1 mL of hybridization buffer, for one hour with gentle mixing. The coated beads were washed 5x with hybridization buffer at 60°C and then resuspended in hybridization buffer at a final concentration of 10 ug/mL.

The magnetic beads were then coated with labeled mouse immunoglobulin by the following procedure: Mouse immunoglobulin (mIgG) was labeled with Sulfo-TAG™ ECL labels (Meso Scale Diagnostics, LLC.) according to the manufacturer's instructions. The protein was also labeled with an oligonucleotide having a terminal thiol group (Oligo 2, the complement of Oligo 1) using a bifunctional coupling reagent (sulfosuccinimidyl 4-(*N*-maléimidométhyl)-1-cyclohexane carboxylate ("SMCC")) and conventional coupling protocols, e.g., protein is reacted with the NHS-ester in SMCC to label the protein and the resulting complex is reacted with thiolated oligonucleotides which reacts with the maleimide group in SMCC. The labeled mlgG-oligo conjugate (0.1 pmol) was then mixed with the oligo-coated magnetic beads (500 ug of beads) in hybridization buffer for 1 hour at room temperature to hybridize the complementary oligonucleotide sequences and thereby immobilize the mIgG onto the beads. The resulting antibody-coated beads were washed and resuspended in hybridization buffer.

The beads were incubated under different conditions, including incubating the suspension at room temperature for one hour (with or without the presence of free Oligo2 as a competitor) and incubating the suspension at 60°C for 10 min. (with or without the presence of free Oligo2 as a competitor). The beads were then magnetically collected and the supernatant analyzed by ECL assay to measure the amount of labeled mIgG that was released from the beads. To measure the labeled mIgG, the supernatant was transferred to the well of a MULTI-ARRAY plate in which the electrode is coated with goat anti-mouse antibodies (MULTI-ARRAY GAM Plate, Meso Scale Diagnostics, LLC.). The plate was incubated with shaking during which time labeled mIgG in the solution bound to the immobilized goat anti-mouse antibodies. The wells were washed with PBS, filled with 150 uL of Read Buffer T (Meso Scale Diagnostics) and analyzed on a SECTOR Imager instrument.

Table 1 shows that, in the absence of competing oligonucleotides, the linkage of the mIgG to the beads was stable at room temperature. The mIgG could be efficiently released from the beads by exposure to short periods of time above the melting temperature of the Oligol-Oligo2 pair. The efficiency of release could be further enhanced by addition of free Oligo2 as a competitor.

**Table 1. Efficiency of different release techniques,**

| **Release Technique** | **% of Released Material** |
|---|---|
| 1 H at RT | 6% |
| 1H at RT with free Oligo | 23% |
| 10 min 60C | 50% |
| 10 min 60 C with Free Oligo | 57% |

### Example 4 - Assay including capture of analyte through collection of magnetic particles and release by denaturation of a linkage comprising an oligonucleotide pair

Magnetic beads (Dynalbeads® MyOne™-Streptavidin C1 beads, Invitrogen Corporation) were coated with biotinylated oligonucleotides as described in Example 3. The magnetic beads were then coated with antibodies against human TNF-alpha and IL-5 using i) antibodies that were labeled with Sulfo-TAG and Oligol and ii) the coating procedure of Example 3.

Assay Procedure with Pre-Concentration. Sample containing human TNF-alpha or IL-5 (1 mL of sample) was combined with 200 ng of antibody-coated beads (prepared as described above) and incubated for 1 hr at room temperature. The beads were magnetically collected and washed with hybridization buffer. The antibody on the beads (including any labeled-antibody-analyte complexes that were formed during the incubation) were released into 100 uL of a 1:20 dilution of hybridization buffer (∼10 mM salt) at elevated temperature (60°C), i.e., by denaturing the oligonucleotide pairs linking the antibodies to the beads. The resulting solution was transferred to a well of a MULTI-ARRAY 96-well plate, each well of which included an array of capture antibodies including an anti-TNF-alpha spot and an anti-IL-5 spot. The plate was incubated with shaking for 1 hr at room temperature to allow the labeled-antibody-analyte complexes to bind to the appropriate capture antibody spots. The wells were then washed three times with PBS and then filled with 125 uL of Read Buffer T (Meso Scale Diagnostics) and read on a SECTOR Imager instrument. The instrument measures and reports the ECL intensity from each array element (or "spot") in the antibody array.

Conventional Immunoassay Protocol without Pre-Concentration. Sample containing human TNF-alpha or IL-5 (30 uL) was combined with 20 uL of a solution containing labeled (Sulfo-TAG) detection antibodies at a concentration of 1 ug/mL. The resulting solution was incubated for 1 hr in a well of a MULTI-ARRAY plate having anti-TNF-alpha and anti-IL-5 spots. The wells were washed, filled with Read Buffer T and analyzed in a SECTOR Imager instrument as described for the protocol with collection and release.

Results. The results presented in Table 2 show that the protocol with collection and release provided specific assay signals for both TNF-alpha and IL-5 (signal in the presence of analyte - signal in the absence of analyte) that were substantially higher than those obtained using the conventional protocol, without any substantial change in the background signal in the absence of analyte. The enhancement in specific signal for 10 pg/mL samples was greater than 5-fold for TNF-alpha and greater than 10-fold for IL-5.

**Table 2.**

| Assay | TNF | | IL-5 | |
|---|---|---|---|---|
| Analyte Concentration, pg/mL | Conventional | Pre-Concentration | Conventional | Pre-Concentration |
| 0 | 371 | 398 | 22 | 27 |
| 1 | 530 | 1,095 | 95 | 451 |
| 10 | 3,301 | 18,323 | 723 | 9,831 |
| 100 | 31,005 | 75,864 | 8,057 | 48,895 |

| | | | | |
|---|---|---|---|---|
| *** | | | | |

The present invention is not to be limited in scope by the specific embodiments described herein. Indeed, various modifications of the method in addition to those described herein will become apparent to those skilled in the art from the foregoing description and accompanying figures. Such modifications are intended to fall within the scope of the claims. Various publications are cited herein, the disclosures of which are incorporated by reference in their entireties.
The current disclosure contains, *inter alia*, the following items:
1. A method of conducting a binding assay comprising
   (a) contacting (i) a sample comprising a target analyte with (ii) a particle linked to a first binding reagent that binds said target analyte and thereby forms a complex comprising said target analyte bound to said first binding reagent;
   (b) collecting said complex;
   (c) separating unbound components of said sample from said complex;
   (d) releasing said complex;
   (e) contacting said complex with a second binding reagent bound to a solid phase, wherein said second binding reagent binds to said complex; and
   (f) measuring the amount of said analyte bound to said solid phase.
2. The method of item 1 wherein said releasing step (d) comprises resuspending said particle.
3. The method of item 1, wherein said releasing step (d) comprises cleaving said first binding reagent from said particle.
4. The method of item 1 wherein said first binding reagent comprises a detectable label.
5. The method of item 1 wherein said second binding reagent comprises a detectable label.
6. The method of item 1 wherein said measuring step comprises contacting said complex with a third binding reagent comprising a detectable label.
7. The method of item 1 wherein said particle comprises a detectable label.
8. The method of item 3 wherein said cleaving step comprises subjecting said complex to increased or decreased temperature, pH changes, competition, and combinations thereof.
9. The method of item 1 wherein said collecting step comprises a method selected from the group consisting of centrifugation, gravity, filtration, magnetic collection, and combinations thereof.
10. The method of item 1 wherein said measuring step comprises measuring optical absorbance, fluorescence, phosphorescence, chemiluminescence, light scattering or magnetism.
11. The method of any one of items 4-7 wherein said detectable label is an ECL label and said measuring step comprises measuring an ECL signal and correlating said signal with an amount of analyte in said sample.
12. The method of item 11 wherein said solid phase is an electrode and said measuring step further comprises applying a voltage waveform to said electrode to generate ECL.
13. The method of item 1 wherein said assay is a sandwich assay or a competitive assay.
14. A method of conducting a binding assay comprising
   (a) contacting (i) a sample comprising a target analyte with (ii) a first solid phase linked to a first binding reagent that binds said target analyte and forms a complex comprising said target analyte bound to said first binding reagent;
   (b) separating unbound components of said sample from said complex;
   (c) releasing said complex;
   (d) separating said complex from said first solid phase;
   (e) contacting said complex with a second binding reagent bound to a second solid phase, wherein said second binding reagent binds to said complex; and
   (f) measuring the amount of said analyte bound to said second solid phase.
15. The method of item 14 wherein said releasing step (c) comprises resuspending said complex.
16. The method of item 14 wherein said releasing step (c) comprises cleaving said first binding reagent from said first solid phase.
17. The method of item 14 wherein said first binding reagent comprises a detectable label.
18. The method of item 14 wherein said second binding reagent comprises a detectable label.
19. The method of item 14 wherein said measuring step comprises contacting said complex with a third binding reagent comprising a detectable label.
20. The method of item 16 wherein said cleaving step comprises subjecting said complex to increased or decreased temperature, pH changes, competition, and combinations thereof.
21. The method of item 14 wherein said collecting step comprises a method selected from the group consisting of centrifugation, gravity, filtration, magnetic collection, and combinations thereof.
22. The method of item 14 wherein said measuring step comprises measuring optical absorbance, fluorescence, phosphorescence, chemiluminescence, light scattering or magnetism.
23. The method of any one of items 17-19 wherein said detectable label is an ECL label and said measuring step comprises measuring an ECL signal and correlating said signal with an amount of analyte in said sample.
24. The method of any one of items 17-19 wherein said solid phase is an electrode and said measuring step further comprises applying a voltage waveform to said electrode to generate ECL.
25. The method of item 14 wherein said assay is a sandwich assay or a competitive assay,
26. A method of conducting a binding assay comprising
   (a) contacting (i) a sample comprising a target analyte with (ii) a particle linked to a first binding reagent that binds said target analyte and thereby forms a complex comprising said target analyte bound to said particle linked-first binding reagent;
   (b) collecting said complex;
   (c) separating unbound components of said sample from said complex;
   (d) releasing said complex by cleaving said first binding reagent from said particle;
   (e) contacting said complex with a second binding reagent bound to a solid phase, wherein said second binding reagent binds to said complex;
   (f) contacting said complex with a third binding reagent comprising a detectable label; and
   (g) measuring the amount of said analyte bound to said solid phase.
27. The method of item 26 wherein said cleaving step comprises subjecting said complex to increased or decreased temperature, pH changes, competition, and combinations thereof.
28. The method of item 26 wherein said collecting step comprises a method selected from the group consisting of centrifugation, gravity, filtration, magnetic collection, and combinations thereof.
29. The method of item 26 wherein said measuring step comprises measuring optical absorbance, fluorescence, phosphorescence, chemiluminescence, light scattering or magnetism.
30. The method item 26 wherein said measuring step comprises measuring an ECL signal emitted by an ECL label linked to a component of said assay and correlating said signal with an amount of analyte in said sample.
31. The method of item 30 wherein said solid phase is an electrode and said measuring step further comprises applying a voltage waveform to said electrode to generate ECL.
32. The method of item 26 wherein said assay is a sandwich assay or a competitive assay.
33. A method of conducting a binding assay for a plurality of analytes comprising
   (a) contacting (i) a sample with (ii) one or more first solid phases linked to one or more first binding reagents that bind said analytes to form complexes comprising said analytes bound to said first binding reagents;
   (b) separating unbound components of said sample from said complexes;
   (c) releasing said complexes;
   (d) contacting said complexes with one or more binding domains comprising second binding reagents, wherein each binding domain comprises a second binding reagent that binds to a complex comprising an analyte; and
   (e) measuring the amount of said analytes bound to said binding domains.
34. The method of item 33 wherein said releasing step (c) comprises resuspending said complexes.
35. The method of item 33 wherein said releasing step (c) comprises cleaving said first binding reagent from said first solid phases.
36. The method of item 33 wherein said first binding reagents comprise a detectable label.
37. The method of item 33 wherein said second binding reagents comprise a detectable label.
38. The method of item 33 wherein said measuring step comprises contacting said complexes with one or more third binding reagents comprising a detectable label.
39. The method of item 35 wherein said cleaving step comprises subjecting said complex to increased or decreased temperature, pH changes, competition, and combinations thereof.
40. The method of item 33 wherein said collecting step comprises a method selected from the group consisting of centrifugation, gravity, filtration, magnetic collection, and combinations thereof.
41. The method of item 33 wherein said measuring step comprises measuring optical absorbance, fluorescence, phosphorescence, chemiluminescence, light scattering or magnetism.
42. The method of item 33 wherein said detectable label is an ECL label and said measuring step comprises measuring an ECL signal and correlating said signal with an amount of analyte in said sample.
43. The method of item 33 wherein said binding domains are located on one or more electrodes and said measuring step further comprises applying a voltage waveform to said electrodes to generate ECL.
44. The method of item 33 wherein said method includes assays selected from the group consisting of sandwich assays, competitive assays, and combinations thereof.
45. A method of conducting a binding assay comprising
   (a) contacting (i) a sample comprising a target analyte with (ii) a particle linked to a first binding reagent that binds said target analyte to form a complex comprising said target analyte bound to said first binding reagent, wherein said first binding reagent is linked to a first targeting agent and said particle is linked to a second targeting agent, and said first binding reagent and said particle are linked via a binding reaction between said first and second targeting agents;
   (b) collecting said complex;
   (c) separating unbound components of said sample from said complex;
   (d) releasing said complex;
   (e) contacting said complex with a second binding reagent bound to a solid phase, wherein said second binding reagent binds to said complex; and
   (f) measuring the amount of said analyte bound to said solid phase.
46. The method of item 45 wherein said releasing step (d) comprises disassociating the binding interaction between said first and second targeting agents.
47. The method of item 45 wherein said releasing step (d) comprises cleaving said first binding reagent from said particle.
48. The method of item 45 wherein said first binding reagent comprises a detectable label.
49. The method of item 45 wherein said second binding reagent comprises a detectable label.
50. The method of item 45 wherein said measuring step comprises contacting said complex with a third binding reagent comprising a detectable label.
51. The method of item 45 wherein said particle comprises a detectable label.
52. The method of item 45 wherein said first and second targeting agents are dissociated by subjecting them to increased or decreased temperature, pH changes, competition, and combinations thereof.
53. The method of item 45 wherein said collecting step comprises a method selected from the group consisting of centrifugation, gravity, filtration, magnetic collection, and combinations thereof.
54. The method of item 45 said measuring step comprises measuring optical absorbance, fluorescence, phosphorescence, chemiluminescence, light scattering or magnetism.
55. The method of any one of items 48-51 wherein said detectable label is an ECL label and said measuring step comprises measuring an ECL signal and correlating said signal with an amount of analyte in said sample.
56. The method of item 45 wherein said solid phase is an electrode and said measuring step further comprises applying a voltage waveform to said electrode to generate ECL.
57. The method of item 45 wherein said assay is a sandwich assay or a competitive assay.
58. A method of conducting a binding assay comprising
   (a) contacting (i) a sample comprising a target analyte with (ii) a first solid phase linked to a first binding reagent that binds said target analyte to form a complex comprising said target analyte bound to said first binding reagent, wherein said first binding reagent is linked to a first targeting agent and said first solid phase is linked to a second targeting agent, and said first binding reagent and said first solid phase are linked via a binding reaction between said first and second targeting agents;
   (b) separating unbound components of said sample from said complex;
   (c) releasing said complex;
   (d) removing said first solid phase;
   (e) contacting said complex with a second binding reagent bound to a second solid phase, wherein said second binding reagent binds to said complex; and
   (f) measuring the amount of said analyte bound to said second solid phase.
59. The method of item 58 wherein said releasing step (c) comprises disassociating the binding interaction between said first and second targeting agents.
60. The method of items 58 wherein said releasing step (c) comprises cleaving said first binding reagent from said first solid phase.
61. The method of item 58 wherein said first binding reagent comprises a detectable label.
62. The method of item 58 wherein said second binding reagent comprises a detectable label.
63. The method of item 58 wherein said measuring step comprises contacting said complex with a third binding reagent comprising a detectable label.
64. The method of item 58 wherein said first and second targeting agents are dissociated by subjecting them to increased or decreased temperature, pH changes, competition, and combinations thereof.
65. The method of item 58 wherein said collecting step comprises a method selected from the group consisting of centrifugation, gravity, filtration, magnetic collection, and combinations thereof.
66. The method of item 58 wherein said measuring step comprises measuring optical absorbance, fluorescence, phosphorescence, chemiluminescence, light scattering or magnetism.
67. The method of any one of items 61-63 wherein said detectable label is an ECL label and said measuring step comprises measuring an ECL signal and correlating said signal with an amount of analyte in said sample.
68. The method of item 58 wherein said solid phase is an electrode and said measuring step further comprises applying a voltage waveform to said electrode to generate ECL.
69. The method of item 58 wherein said assay is a sandwich assay, or a competitive assay.
70. A method of conducting a binding assay comprising
   (a) contacting (i) a sample comprising a target analyte with (ii) a particle linked to a first binding reagent that binds said target analyte to form a complex comprising said target analyte bound to said first binding reagent, wherein said first binding reagent is linked to a first targeting agent and said particle is linked to a second targeting agent, and said first binding reagent and said particle are linked via a binding reaction between said first and second targeting agents;
   (b) collecting said complex;
   (c) separating unbound components of said sample from said complex;
   (d) releasing said complex by
      cleaving said first binding reagent from said particle;
   (e) contacting said complex with a second binding reagent bound to a solid phase, wherein said second binding reagent binds to said complex;
   (f) contacting said complex with a third binding reagent comprising a detectable label; and
   (g) measuring the amount of said analyte bound to said solid phase.
71. The method of item 70 wherein said cleaving step comprises subjecting said complex to increased or decreased temperature, pH changes, competition, and combinations thereof.
72. The method of item 70 wherein said collecting step comprises a method selected from the group consisting of centrifugation, gravity, filtration, magnetic collection, and combinations thereof.
73. The method of item 70 wherein said measuring step comprises measuring optical absorbance, fluorescence, phosphorescence, chemiluminescence, light scattering or magnetism.
74. The method of item 70 wherein said measuring step comprises measuring an ECL signal emitted by an ECL label linked to a component of said assay and correlating said signal with an amount of analyte in said sample.
75. The method of item 70 wherein said solid phase is an electrode and said measuring step further comprises applying a voltage waveform to said electrode to generate ECL.
76. The method of item 70 wherein said assay is a sandwich assay or a competitive assay.
77. A method of conducting a binding assay for a plurality of analytes comprising
   (a) contacting (i) a sample with (ii) one or more first solid phases linked to one or more first binding reagents that bind said analytes to form complexes comprising said analytes bound to said first binding reagents, wherein said first binding reagents are linked to one or more first targeting agents and said first solid phases are linked to one or more second targeting agents, and said first binding reagents and said first solid phases are linked via binding reactions between said first and second targeting agents;
   (b) separating unbound components of said sample from said complexes;
   (c) releasing said complexes;
   (d) removing said first solid phases;
   (e) contacting said complexes with one or more binding domains comprising second binding reagents, wherein each binding domain comprises a second binding reagent that binds to a complex comprising an analyte; and
   (f) measuring the amount of said analytes bound to said binding domains.
78. The method of item 77 wherein said releasing step (c) comprises disassociating said binding interaction between said first and second targeting agents.
79. The method of item 77 wherein said releasing step (c) comprises cleaving said first binding reagents from said first solid phases.
80. The method of item 77 wherein said first binding reagents comprise a detectable label.
81. The method of item 77 wherein said second binding reagents comprise a detectable label.
82. The method of item 77 wherein said measuring step comprises contacting said complexes with one or more third binding reagents comprising a detectable label.
83. The method of item 77 wherein said cleaving step comprises subjecting said complex to increased or decreased temperature, pH changes, competition, and combinations thereof.
84. The method of item 77 wherein said collecting step comprises a method selected from the group consisting of centrifugation, gravity, filtration, magnetic collection, and combinations thereof.
85. The method of item 77 wherein said measuring step comprises measuring optical absorbance, fluorescence, phosphorescence, chemiluminescence, light scattering or magnetism.
86. The method of any one of items 80-82 wherein said detectable label is an ECL label and said measuring step comprises measuring an ECL signal and correlating said signal with an amount of analyte in said sample.
87. The method of item 77 wherein said binding domains are located on one or more electrodes and said measuring step further comprises applying a voltage waveform to said electrodes to generate ECL.
88. The method of item 77 wherein said method includes assays selected from the group consisting of sandwich assays, competitive assays, and combinations thereof.

## Claims

1. A method of conducting a binding assay for a plurality of analytes comprising
(a) contacting (i) a sample with (ii) one or more first solid phases linked to one or more first binding reagents that bind said analytes to form complexes comprising said analytes bound to said first binding reagents;
(b) separating unbound components of said sample from said complexes;
(c) releasing said complexes;
(d) contacting said complexes with one or more binding domains comprising second binding reagents, wherein each binding domain comprises a second binding reagent that binds to a complex comprising an analyte; and
(e) measuring the amount of said analytes bound to said binding domains.

2. A method of conducting a binding assay comprising
(a) contacting (i) a sample comprising a target analyte with (ii) a particle linked to a first binding reagent that binds said target analyte and thereby forms a complex comprising said target analyte bound to said first binding reagent;
(b) collecting said complex;
(c) separating unbound components of said sample from said complex;
(d) releasing said complex;
(e) contacting said complex with a second binding reagent bound to a solid phase, wherein said second binding reagent binds to said complex; and
(f) measuring the amount of said analyte bound to said solid phase.

3. A method of conducting a binding assay comprising
(a) contacting (i) a sample comprising a target analyte with (ii) a particle linked to a first binding reagent that binds said target analyte and thereby forms a complex comprising said target analyte bound to said particle linked-first binding reagent;
(b) collecting said complex;
(c) separating unbound components of said sample from said complex;
(d) releasing said complex by cleaving said first binding reagent from said particle;
(e) contacting said complex with a second binding reagent bound to a solid phase, wherein said second binding reagent binds to said complex;
(f) contacting said complex with a third binding reagent comprising a detectable label; and
(g) measuring the amount of said analyte bound to said solid phase.

4. A method of conducting a binding assay comprising
(a) contacting (i) a sample comprising a target analyte with (ii) a particle linked to a first binding reagent that binds said target analyte to form a complex comprising said target analyte bound to said first binding reagent, wherein said first binding reagent is linked to a first targeting agent and said particle is linked to a second targeting agent, and said first binding reagent and said particle are linked via a binding reaction between said first and second targeting agents;
(b) collecting said complex;
(c) separating unbound components of said sample from said complex;
(d) releasing said complex;
(e) contacting said complex with a second binding reagent bound to a solid phase, wherein said second binding reagent binds to said complex; and
(f) measuring the amount of said analyte bound to said solid phase.

5. A method of conducting a binding assay comprising
(a) contacting (i) a sample comprising a target analyte with (ii) a first solid phase linked to a first binding reagent that binds said target analyte to form a complex comprising said target analyte bound to said first binding reagent, wherein said first binding reagent is linked to a first targeting agent and said first solid phase is linked to a second targeting agent, and said first binding reagent and said first solid phase are linked via a binding reaction between said first and second targeting agents;
(b) separating unbound components of said sample from said complex;
(c) releasing said complex;
(d) removing said first solid phase;
(e) contacting said complex with a second binding reagent bound to a second solid phase, wherein said second binding reagent binds to said complex; and
(f) measuring the amount of said analyte bound to said second solid phase.

6. A method of conducting a binding assay comprising
(a) contacting (i) a sample comprising a target analyte with (ii) a particle linked to a first binding reagent that binds said target analyte to form a complex comprising said target analyte bound to said first binding reagent, wherein said first binding reagent is linked to a first targeting agent and said particle is linked to a second targeting agent, and said first binding reagent and said particle are linked via a binding reaction between said first and second targeting agents;
(b) collecting said complex;
(c) separating unbound components of said sample from said complex;
(d) releasing said complex by
cleaving said first binding reagent from said particle;
(e) contacting said complex with a second binding reagent bound to a solid phase, wherein said second binding reagent binds to said complex;
(f) contacting said complex with a third binding reagent comprising a detectable label; and
(g) measuring the amount of said analyte bound to said solid phase.

7. A method of conducting a binding assay for a plurality of analytes comprising
(a) contacting (i) a sample with (ii) one or more first solid phases linked to one or more first binding reagents that bind said analytes to form complexes comprising said analytes bound to said first binding reagents, wherein said first binding reagents are linked to one or more first targeting agents and said first solid phases are linked to one or more second targeting agents, and said first binding reagents and said first solid phases are linked via binding reactions between said first and second targeting agents;
(b) separating unbound components of said sample from said complexes;
(c) releasing said complexes;
(d) removing said first solid phases;
(e) contacting said complexes with one or more binding domains comprising second binding reagents, wherein each binding domain comprises a second binding reagent that binds to a complex comprising an analyte; and
(f) measuring the amount of said analytes bound to said binding domains.
